# EUROPEAN PATENT APPLICATION

(11) **EP 2 371 963 A1**
(43) Date of publication of application: **05.10.2011**
(21) Application number: 10158236.9
(22) Date of filing: 29.03.2010
(51) Int. Cl.: C12N 15/80

(54) **Protein production in filamentous fungi**

(71) Applicant: Heinrich-Heine-Universität Düsseldorf, 40225 Düsseldorf (DE)
(72) Inventor: The designation of the inventor has not yet been filed
(74) Representative: Schiweck, Weinzierl & Koch

(57) **Abstract**

The present invention relates to an expression cassette (also referred to herein sometimes as "expression system" or "system") comprising (a) a first nucleotide sequence encoding a polypeptide which is secreted from a fungal host cell; (b) a second nucleotide sequence encoding a polypeptide of interest; and (c) one or more third nucleotide sequences which can be bound by a polypeptide comprising at least one sequence specific RNA binding domain, wherein the order (5'→ 3') of said first (a), second (b) and third (c) nucleotide sequence(s) is as follows: (i) (a), (b) and (c); (ii) (a), (c) and (b); (iii) (b), (a) and (c); (iv) (b), (c) and (a); (v) (c), (a) and (b), or (vi) (c), (b) and (a), wherein the nucleotide sequence of (a) and (b) or (b) and (a) are fused in frame. Also contemplated are vectors comprising the expression cassette, host cells comprising the vectors as well as methods and uses for the production of a polypeptide of interest.

## Description

The present invention relates to an expression cassette (also referred to herein sometimes as "expression system" or "system") comprising (a) a first nucleotide sequence encoding a polypeptide which is secreted from a fungal host cell; (b) a second nucleotide sequence encoding a polypeptide of interest; and (c) one or more third nucleotide sequences which can be bound by a polypeptide comprising at least one sequence specific RNA binding domain, wherein the order (5'→ 3') of said first (a), second (b) and third (c) nucleotide sequence(s) is as follows: (i) (a), (b) and (c); (ii) (a), (c) and (b); (iii) (b), (a) and (c); (iv) (b), (c) and (a); (v) (c), (a) and (b), or (vi) (c), (b) and (a), wherein the nucleotide sequence of (a) and (b) or (b) and (a) are fused in frame. Also contemplated are vectors comprising the expression cassette, host cells comprising the vectors as well as methods and uses for the production of a polypeptide of interest.

Protein expression systems are widely applied in the life sciences, biotechnology and medicine. Molecular biology research uses an enormous number of proteins and enzymes many of which are from expression systems; particularly DNA polymerase for PCR, reverse transcriptase for RNA analysis and restriction endonucleases for cloning. There are also significant medical applications for expression systems, notably the production of antibodies and therapeutic proteins such as human insulin to treat diabetes or vaccines to immunize humans against viral infections such as hepatitis B.

Thus, in particular the demand for the efficient production of biologics for therapeutic use is steadily increasing as more products, such as recombinant proteins, are approved or are nearing approval for use in humans. Bacterial, fungal or mammalian cell expression systems have long been, and still are, the major tool for production of these types of molecules. The key objective of process optimization is to attain a high yield of product having the required quality at the lowest possible cost, which is often determined by the properties of a specific expression construct or system. For example, high-level recombinant protein expression may overwhelm the metabolic capacity of a host cell, which often impairs efficient protein production. It is also known that sometimes high expression of an mRNA encoding a protein of interest does not necessarily lead to high amounts of the protein. Different approaches have been taken by scientists to deal with these problems.

Once expressed by a host cell, a protein of interest is either isolated by lysis or any other measure applied to break the cell membrane and/or cell wall of a host cell and subsequently purified. However, a convenient way to harvest a protein of interest is its isolation from the culture medium. In that case, the protein of interest must be secreted by the host. Secretion of a protein is usually achieved by the use of signal sequences. Specifically, proteins equipped with a signal sequence are secreted through the conventional endoplasmic reticulum (eR)-Golgi secretory pathway, i.e., the conventional secretion pathway. However, some proteins, for example, cytoplasmic, nuclear and signal-peptide-containing proteins have been shown to reach the cell surface by nonconventional transport pathways. The mechanisms and molecular components of unconventional protein secretion are beginning to emerge. Unconventional protein secretion may have some advantages vis-à-vis conventional protein secretion: i) proteins subject to unconventional secretion are not processed by eR or Golgi-dependent post-translational modifications and ii) no disulfide bonds are formed (Rubartelli et al. (1990), EMBO J. 9:1503-1510, Muesch, et al. (1990), TIBS 15: 86-88, Hughes (1999), Biochim Biophys Acta: 1473:172-185).

Particularly the first feature which pertains to unconventional protein secretion may be of high interest since yeasts and fungi usually exert high-mannose type glycosylation to proteins expressed in these organisms. Yet, highly glycosylated proteins are immunogenic to mammals and, thus, yeasts and fungi were manipulated to lack the key enzyme which initiates high-mannose glycosylation, i.e., α 1,6-mannosyltransferase encoded by the och1 gene. However, the deletion of the och1 gene causes growth defects and, thus, the so-manipulated cells may no longer be high-producer strains.

The second property of unconventional protein secretion may be interest since over-expressed proteins have a tendency to form disulfide bonds which may lead to aggregates. Accordingly, the circumvention of the conventional secretion pathway through the eR whose lumen constitutes an oxidizing milieu for proteins may be advantageous to obtain "native" proteins which have free thiol groups that can be used for further manipulation such as targeted pegylation.

The existence of fungal strains producing very high levels of secreted proteins stimulated interest in the use of fungi as hosts for the expression of recombinant proteins. Despite the attention that protein secretion in fungi has attracted, and the multifaceted importance of the process, the understanding of the cellular mechanisms involved is still minimal and, for the most part, it is necessary to extrapolate from other eukaryotic organisms. However, current research suggests that protein secretion in filamentous fungi is intimately associated with the process of growth at the hyphal tip. Apart from the fact that conventional protein secretion is studied in fungi, also unconventional protein secretion is known, for example, from the α-factor of yeast. Thus, fungi, in particular filamentous fungi are an interesting tool for protein production, particularly since they have efficient means to secrete proteins via the conventional and/or unconventional secretion pathway. As such, these fungi may have some exploitable advantages over so far known yeast-based protein expression systems.

Hence, a need exists for identifying and developing additional/alternative protein expression systems useful for the efficient and stable production and secretion of proteins. The present invention meets such needs, and further provides other related advantages. Accordingly, the present invention thus provides as a solution to the technical problem the embodiments concerning expression cassettes, vectors, host cells, kits and uses for the expression of proteins. These embodiments are characterized and described herein, illustrated in the Examples, and reflected in the claims.

It must be noted that as used herein, the singular forms "a", "an", and "the", include plural references unless the context clearly indicates otherwise. Thus, for example, reference to "an expression cassette" includes one or more of the expression cassettes disclosed herein and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".
When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

The present inventor observed that microtubule-dependent mRNA transport is important for polar growth of infectious filaments in the filamentous basidiomycete Ustilago maydis. Combining in vivo UV crosslinking and RNA live imaging revealed that Rrm4 forms an integral part of the mRNP transport machinery and mediates transport of distinct mRNAs. In particular, it was observed that proteins involved in cell wall remodelling, translation and mitochondrial function were differentially regulated in rrm4Δ strains. Thus, the Rrm4, a RNA-binding protein predicted to bind CA-rich sequences specifically, constitutes a key component of this molecular transport system. Focusing in particular on the bacterial-type endochitinase Cts1 in rrmΔ strains showed that Cts1 mRNA is apparently a direct target of Rrm4 and the encoded protein accumulated predominantly at the filament apex (tip) of rrm4Δ strains, while in wild-type cells it is, after its transport to the hyphal tip, secreted predominantly via the unconventional secretion pathway. A direct target may nevertheless include that Cts1 is secreted by the aid of export proteins such as, for example, Ste6p in S. cerevisiae. The mRNA of these export proteins may thus also be transported by Rrm4 and, once translated these export proteins could aid to secerete Cts1. Thus, Rrm4 is apparently crucial for the transport of al least cts1 mRNA and plays an essential role in the efficient secretion of Cts1.
Thus, the present inventor uncovered a novel molecular link of microtubule-dependent mRNA transport and protein secretion in fungi at the hyphal tip.
In sum, the present inventor found that filamentous fungi apparently make use of a so-called "localized translation", i.e., an mRNA (such as cts1 mRNA) is actively transported along microtubules to the site of secretion (i.e. to the hyphal tip), while it is assumed to be translated during its microtubule-dependent transport and/or at the tip of a hypha to get secreted.

As a result, the present invention brings together mRNA transport, translation during transport to the hyphal tip and/or at the hyphal tip and the subsequent secretion of a nascent protein, preferably secretion via the unconventional secretion pathway. In fact, it is known that protein secretion in filamentous fungi is intimately associated with the process of growth at the hyphal tip (Peberdy (1994), Trends Biotechnol. 12:50-57). Unravelling this concept, i.e. making technically available an intrinsic fungal system for efficient protein production and secretion paves the way for industrially exploiting production and secretion (in particular secretion via the unconventional secretion pathway) of a protein of interest in a filamentous fungus. Without being bound by theory, this system has certain advantages: if secreted via the unconventional secretion pathway the protein of interest does not enter the eR and Golgi to become processed, but is translated on its way to get secreted. Thus, proteins without post-translational modifications can be obtained. Moreover, proteins without disulfide bonds are formed and secreted. In addition, the microtubule-dependent transport of the mRNA encoding the protein of interest ensures that the then-translated protein is locally present at an active growth site, i.e. at the tip of a hypha to become efficiently secreted, since fungal hypha are known to secrete many proteins at this active site of growth. Hence, in contrast to "conventional" expression systems, the system of the invention allows a localized translation and secretion and may thus not burden the host cell with overwhelming the metabolic capacity of that host cell, which often impairs efficient protein production.

Accordingly, in a first aspect the present invention relates to an expression cassette (also referred to herein sometimes as "expression system" or "system") comprising:
(a) a first nucleotide sequence encoding a polypeptide which is secreted from a fungal host cell;
(b) a second nucleotide sequence encoding a polypeptide of interest; and
(c) one or more third nucleotide sequences which can be bound by a polypeptide comprising at least one sequence specific RNA binding domain,
   wherein the order (5'→ 3') of said first (a), second (b) and third (c) nucleotide sequence(s) is as follows:
   (i) (a), (b) and (c);
   (ii) (a), (c) and (b),
   (iii) (b), (a) and (c),
   (iv) (b), (c) and (a),
   (v) (c), (a) and (b), or
   (vi) (c), (b) and (a),
   wherein the nucleotide sequence of (a) and (b) or (b) and (a) are fused in frame.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well known in the art. Generally, nomenclatures used in connection with, and techniques of biochemistry, enzymology, molecular and cellular biology, microbiology, genetics and protein and nucleic acid chemistry and hybridization described herein are those well- known and commonly used in the art.

The methods and techniques of the present invention are generally performed according to conventional methods well-known in the art and as described in various general and more specific references that are cited and discussed throughout the present specification unless otherwise indicated. See, e. g., Sambrook et al., Molecular Cloning: A Laboratory Manual, 3rd ed., Cold Spring Harbor Laboratory Press, Cold Spring Harbor, N. Y. (2001); Ausubel et al., Current Protocols in Molecular Biology, J, Greene Publishing Associates (1992, and Supplements to 2002); Handbook of Biochemistry: Section A Proteins, Vol I 1976 CRC Press; Handbook of Biochemistry: Section A Proteins, Vol II 1976 CRC Press. The nomenclatures used in connection with, and the laboratory procedures and techniques of, molecular and cellular biology, protein biochemistry, enzymology and medicinal and pharmaceutical chemistry described herein are those well known and commonly used in the art.

The expression cassettes of the invention that are preferably present in a vector, preferably an expression vector, are designed such that they allow the expression of the incorporated nucleic acid molecule in fungal host cells, preferably fungal host cells which are capable of filamentous growth in liquid culture. For this purpose the expression cassettes usually comprise the necessary regulatory sequences, such as a promoter and/or a transcription termination sequence such as a poly A site.

Any preferred restriction endonuclease site may be incorporated into the expression cassette and/or vector of the invention as described herein below in more detail (see list of commercially available restriction endonucleases in the New England Biolabs catalog, which is hereby incorporated by reference). Preferably, the expression cassette comprises at least one restriction enzyme recognition site at about the 3'-end and at least one restriction enzyme recognition site at about the 5'-end.

As used herein, an "expression cassette" refers to a contiguous nucleic acid molecule that can preferably be isolated as a single unit and cloned as a single functional expresion unit. A functional expression unit, capable of properly driving the expression of an incorporated polynucleotide is thus also referred to as an "expression cassette" herein.

For example, a sequence cassette may be created enzymatically (e.g., by using type I or type II restriction endonucleases, exonucleases, etc.), by mechanical means (e.g., shearing), by chemical synthesis, or by recombinant methods (e.g., PCR). Expression cassettes generally include the following elements (presented in the 5 '-3' direction of transcription): a transcriptional and translational initiation region, a coding sequence for a gene of interest, and a transcriptional and translational termination region functional in the organism where it is desired to express the gene of interest. The expression cassette of the invention comprises at least three elements:
(a) a first nucleotide sequence encoding a polypeptide which is secreted from a fungal host cell;
(b) a second nucleotide sequence encoding a polypeptide of interest; and
(c) one or more third nucleotide sequences which can be bound by a polypeptide comprising at least one sequence specific RNA binding domain.

These three elements (i.e. nucleotide sequences) may be set up in different orders (5'→ 3') as follows:
(i) (a), (b) and (c);
(ii) (a), (c) and (b),
(iii) (b), (a) and (c),
(iv) (b), (c) and (a),
(v) (c), (a) and (b), or
(vi) (c), (b) and (a),
wherein the nucleotide sequence of (a) and (b) or (b) and (a) are fused in frame.

The first nucleotide sequence is also referred to herein as "nucleotide sequence (a)" or simply "(a)". Likewise, the second nucleotide sequence and third nucleotide sequence, respectively, is also referred to herein as "nucleotide sequence (b)" or simply "(b)" and "nucleotide sequence (c)" or simply "(c)".

The terms " 5' " and " 3' " is a convention used to describe features of a nucleotide sequence related to either the position of genetic elements and/or the direction of events (5' to 3'), such as e.g. transcription by RNA polymerase or translation by the ribosome which proceeds in 5' to 3' direction. Synonyms are upstream (5') and downstream (3'). Conventionally, nucleotide sequences, gene maps, vector cards and RNA sequences are drawn with 5' to 3' from left to right or the 5' to 3' direction is indicated with arrows, wherein the arrowhead points in the 3' direction. Accordingly, 5' (upstream) indicates genetic elements positioned towards the left hand side, and 3' (downstream) indicates genetic elements positioned towards the right hand side, when following this convention.

The term "nucleotide sequence" or" nucleic acid molecule" refers to a polymeric form of nucleotides (i.e. polynucleotide) of at least 10 bases in length which are usually linked from one deoxyribose or ribose to another. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The term "nucleotide sequence" does not comprise any size restrictions and also encompasses nucleotides comprising modifications, in particular modified nucleotides, e.g., as described herein.

In this regard, a nucleic acid being an expression product is preferably a RNA, whereas a nucleic acid to be introduced into a cell is preferably DNA.

The nucleic acid can be in any topological conformation. For instance, the nucleic acid can be single-stranded, double-stranded, triple-stranded, quadruplexed, partially double-stranded, branched, hairpinned, circular, or in a padlocked conformation.

The term "nucleotide sequence" includes single and double stranded forms of DNA. A nucleic acid molecule of this invention may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The nucleotide sequences of the invention are preferably "isolated" or "substantially pure". An "isolated" or "substantially pure" nucleotide sequence or nucleic acid (e.g., a RNA, DNA or a mixed polymer) is one which is substantially separated from other cellular components that naturally accompany the native polynucleotide in its natural host cell, e.g., ribosomes, polymerases, and genomic sequences with which it is naturally associated. The term embraces a nucleotide sequence or nucleic acid that (1) has been removed from its naturally occurring environment, (2) is not associated with all or a portion of a polynucleotide in which the "isolated nucleotide sequence" is found in nature, (3) is operatively linked to a polynucleotide which it is not linked to in nature, or (4) does not occur in nature. The term "isolated" or "substantially pure" also can be used in reference to recombinant or cloned DNA isolates, chemically synthesized polynucleotide analogs, or polynucleotide analogs that are biologically synthesized by heterologous systems.

However, "isolated" does not necessarily require that the nucleotide sequence or nucleic acid so described has itself been physically removed from its native environment. For instance, an endogenous nucleotide sequence in the genome of an organism is deemed "isolated" herein if a heterologous sequence (i.e., a sequence that is not naturally adjacent to this endogenous nucleic acid sequence) is placed adjacent to the endogenous nucleic acid sequence, such that the expression of this endogenous nucleic acid sequence is altered. By way of example, a non- native promoter sequence can be substituted (e.g., by homologous recombination) for the native promoter of a gene in the genome of a human cell, such that this gene has an altered expression pattern. This gene would now become "isolated" because it is separated from at least some of the sequences that naturally flank it.

A nucleotide sequence is also considered "isolated" if it contains any modifications that do not naturally occur to the corresponding nucleic acid in a genome. For instance, an endogenous coding sequence is considered "isolated" if it contains an insertion, deletion or a point mutation introduced artificially, e.g., by human intervention. An "isolated nucleotide sequence" includes a nucleic acid integrated into a host cell chromosome at a heterologous site, a nucleic acid construct present as an episome. Moreover, an "isolated nucleotide sequence" can be substantially free of other cellular material, or substantially free of culture medium when produced by recombinant techniques, or substantially free of chemical precursors or other chemicals when chemically synthesized.

When used herein, the phrase "degenerate variant" of a reference nucleotide sequence encompasses nucleotide sequences that can be translated, according to the standard genetic code, to provide an amino acid sequence identical to that translated from the reference nucleotide sequence.

Unless otherwise indicated, a "nucleotide sequence shown in SEQ ID No:X" refers to a nucleotide sequence, at least a portion of which has either (i) the sequence of SEQ ID No:X, or (ii) a sequence complementary to SEQ ID No:X. The choice between the two is dictated by the context. For instance, if the nucleic acid is used as a probe, the choice between the two is dictated by the requirement that the probe be complementary to the desired target.

A "polypeptide" refers to a molecule comprising a polymer of amino acids linked together by a peptide bond(s). Said term is herein interchangeably used with the term "protein". When used herein, the term "polypeptide" or "protein" also includes a "polypeptide of interest" or "protein of interest" which is expressed by the expression cassettes or vectors or can be isolated from the host cells of the invention.

A "polypeptide" as used herein encompasses both naturally-occurring and non-naturally-occurring proteins, and fragments, mutants, derivatives and analogs thereof. Polypeptides include polypeptides and peptides of any length, including proteins (for example, having more than 50 amino acids) and peptides (for example, having 2-10, 2-20, 2-30, 2-40 or 2-49 amino acids). Polypeptides include proteins and/or peptides of any activity or bioactivity. A "peptide" encompasses analogs and mimetics that mimic structural and thus biological function.

Polypeptides may further form dimers, trimers and higher oligomers, i.e. consisting of more than one polypeptide molecule. Polypeptide molecules forming such dimers, trimers etc. may be identical or non-identical. The corresponding higher order structures are, consequently, termed homo- or heterodimers, homo- or heterotrimers etc. The terms "polypeptide" and "protein" also refer to naturally or non-naturally modified polypeptides/proteins wherein the modification is effected e.g. by glycosylation, acetylation, phosphorylation and the like. Such modifications are well known in the art.

Further, a polypeptide may comprise a number of different domains each of which has one or more distinct activities.

The term "isolated protein" or "isolated polypeptide" is a protein or polypeptide that by virtue of its origin or source of derivation (1) is not associated with naturally associated components that accompany it in its native state, (2) when it exists in a purity not found in nature, where purity can be adjudged with respect to the presence of other cellular material (e.g. , is free of other proteins from the same species) (3) is expressed by a cell from a different species, or (4) does not occur in nature (e.g. , it is a fragment of a polypeptide found in nature or it includes amino acid analogs or derivatives not found in nature or linkages other than standard peptide bonds). Thus, a polypeptide that is chemically synthesized or synthesized in a cellular system different from the cell from which it naturally originates will be"isolated"from its naturally associated components. A polypeptide or protein may also be rendered substantially free of naturally associated components by isolation, using protein purification techniques well- known in the art. As thus defined,"isolated" does not necessarily require that the protein, polypeptide, peptide or oligopeptide so described has been physically removed from its native environment.

The term "polypeptide fragment" or "fragment" of a polypeptide as used herein refers to a polypeptide that has an amino-terminal and/or carboxy-terminal deletion compared to a full- length polypeptide. In a preferred embodiment, the polypeptide fragment is a contiguous sequence in which the amino acid sequence of the fragment is identical to the corresponding positions in the naturally-occurring sequence. Fragments typically are at least 5, 6, 7, 8, 9 or 10 amino acids long, preferably at least 12, 14, 16 or 18 amino acids long, more preferably at least 20 amino acids long, more preferably at least 25, 30, 35, 40 or 45, amino acids, even more preferably at least 50 or 60 amino acids long, and even more preferably at least 70 amino acids long. Fragments have preferably the same biological activity as the full-length polypeptide.

A "modified derivative" refers to polypeptides or fragments thereof that are substantially homologous in primary structural sequence but which include, e. g., in vivo or in vitro chemical and biochemical modifications or which incorporate amino acids that are not found in the native polypeptide. Such modifications include, for example, acetylation, carboxylation, phosphorylation, glycosylation, ubiquitination, labeling, e.g., with radionuclides, and various enzymatic modifications, as will be readily appreciated by those well skilled in the art. A variety of methods for labeling polypeptides and of substituents or labels useful for such purposes are well-known in the art, and include radioactive isotopes such as ¹²⁵I, ³²P, ³⁵S, and ³H, ligands which bind to labeled antiligands (e.g., antibodies), fluorophores, chemiluminescent agents, enzymes, and antiligands which can serve as specific binding pair members for a labeled ligand. The choice of label depends on the sensitivity required, ease of conjugation with the primer, stability requirements, and available instrumentation. Methods for labeling polypeptides are well-known in the art.

A "polypeptide mutant" or "mutein" refers to a polypeptide whose sequence contains an insertion, duplication, deletion, rearrangement or substitution of one or more amino acids compared to the amino acid sequence of a native or wild type protein. A mutein may have one or more amino acid point substitutions, in which a single amino acid at a position has been changed to another amino acid, one or more insertions and/or deletions, in which one or more amino acids are inserted or deleted, respectively, in the sequence of the naturally-occurring protein, and/or truncations of the amino acid sequence at either or both the amino or carboxy termini. A mutein may have the same but preferably has a different biological activity compared to the naturally-occurring protein. For example, mutein of the polypeptide encoded by nucleotide sequence (a) and/or (b) is envisaged to be comprised by the expression cassette of the invention.

A mutein has at least 70% overall sequence homology to its wild-type counterpart. Even more preferred are muteins having 80%, 85% or 90% overall sequence homology to the wild-type protein. In an even more preferred embodiment, a mutein exhibits 95% sequence identity, even more preferably 97%, even more preferably 98% and even more preferably 99% overall sequence identity. Sequence homology may be measured by any common sequence analysis algorithm, such as Gap or Bestfit.

Preferred amino acid substitutions are those which: (1) reduce susceptibility to proteolysis, (2) reduce susceptibility to oxidation, (3) alter binding affinity for forming protein complexes, (4) alter binding affinity or enzymatic activity, and (5) confer or modify other physicochemical or functional properties of such analogs.

As used herein, the twenty conventional amino acids and their abbreviations follow conventional usage. See Immunology-A 4 Synthesis (2nd Edition, E. S. Golub and D. R. Gren, Eds., Sinauer Associates, Sunderland, Mass. (1991)). Stereoisomers (e.g., D-amino acids) of the twenty conventional amino acids, unnatural amino acids such as a, a-disubstituted amino acids, N-alkyl amino acids, and other unconventional amino acids may also be suitable components for polypeptides of the present invention.

Examples of unconventional amino acids include: 4-hydroxyproline, Y-carboxyglutamate,-N , N, N-trimethyllysine, E-N-acetyllysine, O-phosphoserine, N-acetylserine, N-formylmethionine, 3-methylhistidine, 5-hydroxylysine, s-N-methylarginine, and other similar amino acids and imino acids (e.g., 4-hydroxyproline). In the polypeptide notation used herein, the left-hand direction is the amino terminal direction and the right hand direction is the carboxy-terminal direction, in accordance with standard usage and convention.

A protein has "homology" or is "homologous" to a second protein if the nucleic acid sequence that encodes the protein has a similar sequence to the nucleic acid sequence that encodes the second protein. Alternatively, a protein has homology to a second protein if the two proteins have"similar"amino acid sequences. Thus, the term "homologous proteins" is defined to mean that the two proteins have similar amino acid sequences). In a preferred embodiment, a homologous protein is one that exhibits at least 60% sequence homology to the wild type protein, more preferred is at least 70% sequence homology. Even more preferred are homologous proteins that exhibit at least 80%, 85% or 90% sequence homology to the wild type protein. In a yet more preferred embodiment, a homologous protein exhibits at least 95%, 97%, 98% or 99% sequence identity. As used herein, homology between two regions of amino acid sequence (especially with respect to predicted structural similarities) is interpreted as implying similarity in function.

When "homologous" is used in reference to proteins or peptides, it is recognized that residue positions that are not identical often differ by conservative amino acid substitutions. A "conservative amino acid substitution" is one in which an amino acid residue is substituted by another amino acid residue having a side chain (R group) with similar chemical properties (e.g., charge or hydrophobicity).

In general, a conservative amino acid substitution will not substantially change the functional properties of a protein. In cases where two or more amino acid sequences differ from each other by conservative substitutions, the percent sequence identity or degree of homology may be adjusted upwards to correct for the conservative nature of the substitution. Means for making this adjustment are well known to those of skill in the art.

The following six groups each contain amino acids that are conservative substitutions for one another: 1) Serine (S), Threonine (T); 2) Aspartic Acid (D), Glutamic Acid (E); 3) Asparagine (N), Glutamine (Q); 4) Arginine (R), Lysine (K); 5) Isoleucine (1), Leucine (L), Methionine (M), Alanine (A), Valine (V), and 6) Phenylalanine (F), Tyrosine (Y), Tryptophan (W).

Sequence homology for polypeptides, which is also referred to as percent sequence identity, is typically measured using sequence analysis software. See, e. g. , the Sequence Analysis Software Package of the Genetics Computer Group (GCG), University of Wisconsin Biotechnology Center, 910 University Avenue, Madison, Wisconsin 53705. Protein analysis software matches similar sequences using measure of homology assigned to various substitutions, deletions and other modifications, including conservative amino acid substitutions. For instance, GCG contains programs such as "Gap" and "Bestfit" which can be used with default parameters to determine sequence homology or sequence identity between closely related polypeptides, such as homologous polypeptides from different species of organisms or between a wild type protein and a mutein thereof. See, e. g., GCG Version 6.1.

A preferred algorithm when comparing a inhibitory molecule sequence to a database containing a large number of sequences from different organisms is the computer program BLAST (Altschul et al. (1990) J Mol. Biol. 215: 403-410; Gish and States (1993) Nature Genet. 3: 266-272; Madden et al. (1996) Meth. Enzymol. 266: 131-141; Altschul et al. (1997) Nucleic Acids Res. 25: 3389- 3402; Zhang and Madden (1997) Genome Res. 7: 649-656), especially blastp or tblastn (Altschul et al., 1997). Preferred parameters for BLASTp are: Expectation value: 10 (default); Filter: seg (default); Cost to open a gap: 11 (default); Cost to extend a gap: 1 (default); Max. alignments: 100 (default); Word size: 11 (default); No. of descriptions: 100 (default); Penalty Matrix: BLOWSUM62.

The length of polypeptide sequences compared for homology will generally be at least about 16 amino acid residues, usually at least about 20 residues, more usually at least about 24 residues, typically at least about 28 residues, and preferably more than about 35 residues. When searching a database containing sequences from a large number of different organisms, it is preferable to compare amino acid sequences. Database searching using amino acid sequences can be measured by algorithms other than blastp known in the art. For instance, polypeptide sequences can be compared using FASTA, a program in GCG Version 6.1. FASTA provides alignments and percent sequence identity of the regions of the best overlap between the query and search sequences (Pearson, 1990, herein incorporated by reference). For example, percent sequence identity between amino acid sequences can be determined using FASTA with its default parameters (a word size of 2 and the PAM250 scoring matrix), as provided in GCG Version 6.1, herein incorporated by reference).

By a "substantially pure polypeptide" is meant any polypeptide which has been separated from naturally accompanying components. Typically, the polypeptide is substantially pure when it is at least 60%, by weight, free from the proteins and naturally-occurring organic molecules with which it is naturally associated. Preferably, the preparation is at least 75%, more preferably at least 90%, and most preferably at least 99%, by weight. A substantially pure polypeptide may be obtained, for example, by extraction from a natural source (such as a cell); by expression of a recombinant nucleic acid encoding the polypeptide; or by chemically synthesizing the protein. Purity can be measured by any appropriate method such as those described in column chromatography, polyacrylamide gel electrophoresis, or by HPLC analysis. A protein is substantially free of naturally associated components when it is separated from those contaminants which accompany it in its natural state. Thus, a protein which is chemically synthesized or produced in a cellular system different from the cell from which it naturally originates will be substantially free from its naturally associated components.

The embodiments and disclosure provided herein with respect to polypeptides/proteins herein also pertain, mutatis mutandis, to the polypeptide of interest produced in accordance with the invention.

A large number of suitable methods exist in the art to produce polypeptides (or fusion proteins) in the host cells of the invention. Conveniently, the produced protein is harvested from the culture medium, lysates of the cultured host cell or from isolated (biological) membranes by established techniques. For example, the expression cassettes as described herein comprising, inter alia, the nucleotide sequence encoding the protein of interest can be synthesized by PCR and inserted into an expression vector. Subsequently a cell produced with the method of the present invention may be transformed with the expression vector. Thereafter, the cell is cultured to produce/express the desired protein(s), which is/are isolated and purified. For example, the product may be recovered from the host cell and/or culture medium by conventional procedures including, but not limited to, cell lysis, breaking up host cells, centrifugation, filtration, ultra-filtration, extraction or precipitation. Purification may be performed by a variety of procedures known in the art including, but not limited to, chromatography (e.g. ion exchange, affinity, hydrophobic, chromatofocusing, and size exclusion), electrophoretic procedures (e.g., preparative isoelectric focusing), differential solubility (e.g. ammonium sulfate precipitation) or extraction.

"Isolating the compound" refers to the separation of the compound produced during or after expression of the nucleic acid introduced. After disintegrating the cells, various separation methods are known in the art. In the case of proteins or peptides as expression products, said proteins or peptides, apart from the sequence necessary and sufficient for the protein to be functional, may comprise additional N- or C- terminal amino acid sequences. Such proteins are referred to as fusion proteins.

Polypeptides produced according to the method of the present invention depict preferably good stability properties. It is envisaged that the polypeptides are expressed in a functional form and hence in the right conformation. Accordingly, the invention also provides polypeptides obtained by the production method according to the present invention using the expression cassette, vector and/or host cell described herein in detail above.

Preferred examples of a polypeptide of interest are enzymes including biocatalysts, receptors, receptor ligands such as competitors and scavenger receptors, antibodies, therapeutic proteins such as interferons, BMPs, GDF proteins, fibroblast growth factors, peptides such as protein inhibitors, membrane proteins, membrane-associated proteins, peptide/protein hormones, cytokines, peptidic toxins, peptidic antitoxins, and the like. It is envisaged that the polypeptide of interest is processed during and/or after its isolation from the culture medium and/or host cell by enzymatic cleavage which is possible, since the expression cassette may, inter alia, contain a nucleotide sequence which encodes a protease cleavage site. Furthermore, it is envisaged that the polypeptide of interest is processed by post-isolation methods such as pegylation, acetylation, phosphorylation, and the like.

When a polypeptide of interest is expressed in a host cell of the invention, it may be necessary to modify the nucleotide sequence encoding said polypeptide by adapting the codon usage of said nucleotide sequence to meet the frequency of the preferred codon usage of said host cell. As used herein, "frequency of preferred codon usage" refers to the preference exhibited by the host cell of the invention in usage of nucleotide codons to specify a given amino acid. To determine the frequency of usage of a particular codon in a gene, the number of occurrences of that codon in the gene is divided by the total number of occurrences of all codons specifying the same amino acid in the gene. Similarly, the frequency of preferred codon usage exhibited by a host cell can be calculated by averaging frequency of preferred codon usage in a large number of genes expressed by the host cell. It is preferable that this analysis be limited to genes that are highly expressed by the host cell. The percent deviation of the frequency of preferred codon usage for a synthetic gene from that employed by a host cell is calculated first by determining the percent deviation of the frequency of usage of a single codon from that of the host cell followed by obtaining the average deviation over all codons. As defined herein, this calculation includes unique codons (i.e., ATG and TGG). In general terms, the overall average deviation of the codon usage of an optimized gene from that of a host cell is calculated using the equation 1A=n=1ZXn-YnXn times 100 Z where Xn=frequency of usage for codon n in the host cell; Yn=frequency of usage for codon n in the synthetic gene; n represents an individual codon that specifies an amino acid; and the total number of codons is Z. The overall deviation of the frequency of codon usage, A, for all amino acids should preferably be less than about 25%, and more preferably less than about 10%.

The term "fused in frame" or "in frame" means that two or more nucleotide sequences are covalently linked together by 5'-3' bonds of the sugar backbone of a nucleic acid such that these two or more nucleotide sequences are in the same open reading frame which is transcribed and then translated as one entity. Accordingly, when the mRNA is transcribed from said covalenty linked nucleic acid and translated a "fusion protein" is formed, since a ribosome translates the mRNA of these two or more nucleotide sequences as if it were one entity, i.e., the mRNA encodes, so to say, one protein, i.e., a fusion protein.

A "fusion protein" thus refers to a polypeptide comprising a first polypeptide or fragment coupled to a second polypeptide or fragment. Fusion proteins are useful because they can be constructed to contain two or more desired functional elements from two or more different proteins. Preferably, fusion proteins can be produced recombinantly in accordance with the invention by constructing a first nucleic acid sequence which encodes the first polypeptide or a fragment thereof in-frame with a second, third, fourth, fifth, etc. nucleic acid sequence encoding a further protein or peptide and then expressing the fusion protein. Alternatively, but less preferred a fusion protein can be produced chemically by crosslinking the polypeptide or a fragment thereof to another protein.

Preferably, in the expression cassette of the invention nucleotide sequence (a) is fused in frame with the nucleotide sequence (b) or vice versa, i.e. the nucleotide sequence (b) is fused in frame with with nucleotide sequence (a). Accordingly, a fusion protein is formed during translation that comprises (N-terminal) a polypeptide which is secreted from a fungal host cell and (C-terminal) a polypeptide of interest; or vice versa, i.e. a fusion protein comprising (N-terminal) a polypeptide of interest and (C-terminal) a polypeptide which is secreted from a fungal host cell.

However, while it is envisaged that nucleotide sequence (a) and (b) or (b) and (a) can be directly fused, i.e., no additional nucleotides are between these nucleotide sequence, nucleotide sequence (a) and (b) or (b) and (a) do not have to be directly fused with eachother, i.e., without additional nucleotides. Thus, the nucleotide sequence (c) can be in between the nucleotide sequence (a) and (b) or (b) and (a). If so. the nucleotide sequence does not need to be in frame with the nucleotide sequence (a) and (b) or (b) and (a). Accordingly, nucleotide sequence (c) can be located 5' and/or 3' of nucleotide sequence (a) and/or (b).

However, nucleotide sequence (c) can preferably be in frame with nucleotide sequence (a) and (b) or (b) and (a). Thus, it is preferred that the the nucleotide sequences (a), (b) and (c) as referred to in (i) to (vi) as described above, are fused in frame.

In yet a further preferred embodiment of the invention, nucleotide sequence(s) (c) is/are comprised in the nucleotide sequence (a) and/or (b).

It is preferred that an expression cassette described herein does not comprise a nucelotide sequence (b) which encodes a β-glucuronidase (GUS). Accordingly, for example, a nucleotide sequence encoding a fusion protein between GUS and Cts1 from *Ustilago maydis* is excluded.

Accordingly, either the nature of the nucleotide sequence (a) and/or (b) is such that it comprises per se, i.e., due to its nucleotide composition one or more nucleotide sequences (c) or the nucleotide sequence (a) and/or (b) is modified such that it then comprises one or more nucleotide sequence(s) (c). For example, the codon usage can be modified by means and methods known in the art or as is described herein elsewhere. Namely, it is known that some of the naturally-occurring amiono acids are encoded by one or more nucleotide triplets and this fact can be exploited when modifying nucleotide sequence (a) and/or (b) so as to then comprise per se one or more nucleotide sequence(s) (c).

In a further preferred aspect of the invention, the expression cassette further comprising one or more (i.e., two, three, four, five, six and more) further nucleotide sequence(s) (c) fused to the 5'- and/or 3'-end of the nucleotide sequence (a) and/or (b). This preferred embodiment, without being bound by theory, may enhance the binding and/or the transport of the resulting transcript (mRNA).

Nucleotide sequence (c) is characterized in that it is bound by a polypeptide comprising at least one sequence specific RNA binding domain. More preferably, the polypeptide which binds nucleotide sequence (c) comprises two, more preferably three, even more preferably four, five, six or more sequence specific RNA binding domains.
A "sequence specific RNA binding domain", when used herein, is a domain of a protein that binds mRNA, in particular a specific sequence of an mRNA. More preferably, a sequence specific RNA binding domain applied in the invention is of the RNA recognition motif (RRM) type. Preferably, an RRM type comprises two tandem RRM and optionally a further RRM separated from the tandem RRM by a hinge region. More preferably, a sequence specific RNA binding domain comprises the following consensus sequence (L/I)(Y/F/I)(L/V/I)XX(V/L) -- 32-46 -(T/K)GX(G/A)FVXF.
Particularly preferred is a sequence specific RNA binding domain comprising the sequence from amino acids 74-368 of SEQ ID No.4.

A preferred polypeptide comprising at least one sequence specific RNA binding domain that is applied in the invention is one which has at least 60%, more preferably at least 70%, even more preferred at least 80%, particularly preferred at least 90% and even more particularly preferred at least 95% identity to the amino acid sequence shown in SEQ ID No.4. A more preferred polypeptide comprising a sequence specific RNA binding domain that is applied in the invention is shown in SEQ ID No.4.

Likewise, a polypeptide comprising at least one sequence specific RNA binding domain that is applied in the invention, can be encoded by a nucleotide sequence which has at least 60%, 70%, 80%, 90% or 95% identity to the nucleotide sequence shown in SEQ ID No:5 or a fragment thereof and which encodes a protein which is capable of sequence specific RNA binding. Alternatively, a nucleotide sequence can be applied which hybridizes to the nucleotide sequence shown in SEQ ID No:5 or a fragment which encodes a protein which is capable of sequence specific RNA binding.

In a preferred aspect, nucleotide sequence (c) bound by a polypeptide comprising at least one sequence specific RNA binding domain comprises one or more (C/A)(C/A)(C/A) repeats, preferably CAA and/or CA, more preferably CA repeats.
In a more preferred embodiment nucleotide sequence (c) comprises the nucleotide sequence shown in SEQ ID No:3 (3' UTR of the ubi1 gene of *Ustilago maydis*). If this sequence is fused in frame with the nucleotide sequence(s) of (a) and/or (b), the skilled person will be aware of the fact that this sequence must not have an "in frame stop codon".

The nucleotide sequence (a) comprises the coding sequence for a polypeptide which is secreted from a fungal host cell. Generally, the expression "coding sequence" refers to the region of continuous sequential DNA triplets encoding a protein, polypeptide or peptide sequence.

Preferably, secretion of a polypeptide from a fungal host cell is via the conventional secretion pathway and/or the unconventional (nonclassical) pathway, the latter being preferred.

The conventional secretion pathway may be summarized as follows: Fungal cells have a highly evolved process of secretion. Proteins targeted for the outside are synthesized by ribosomes docked to the rough endoplasmic reticulum (eR). As they are synthesized, these proteins translocate into the ER lumen, where they are glycosylated and where molecular chaperones aid protein folding. Misfolded proteins are usually identified here and retrotranslocated by ER-associated degradation to the cytosol, where they are degraded by a proteasome. The vesicles containing the properly-folded proteins then enter the Golgi apparatus. In the Golgi apparatus, the glycosylation of the proteins is modified and further posttranslational modifications, including cleavage and functionalization, may occur. The proteins are then moved into secretory vesicles which may travel along the cytoskeleton to the edge of the cell. More modification can occur in these secretory vesicles.

Proteins that are secreted via the conventional secretion pathway usually have a signal-peptide either at their N- or C-terminus. Fungal signal-peptides are known and can be determined, for example, by SignalP (Bendtsen et al., (2004), Protein Eng Des Sel 17: 349-356; Bendtsen, et al. (2004), J. Mol. Biol. 340: 783-795). SignalP 3.0 server is a software tool for predicting signal peptide and cleavage sites in Gram-positive and Gram-negative prokaryotes, and eukaryotic amino acid sequences. Provided on this site are instructions, output format, data sets, abstracts of papers the method is described in, and important notes.

There are many proteins which do not have a signal sequence as determined, for example, by SignalP, SecretomeP, TargetP or Protocomp or by all of these algorithms, yet with SignalP, SecretomeP (Bendtsen et al., (2004), Protein Eng Des Sel 17: 349-356; Bendtsen, et al. (2004), J. Mol. Biol. 340: 783-795), and Protocomp being preferred (www.softberry.de). Protocomp predicts a potential extracellular location of a protein and SecretomeP predicts nonclassical protein secretion.

SecretomeP is a software tool for the prediction of mammalian secretory proteins targeted to the non-classical secretory pathway; proteins without an N-terminal signal peptide. This tool is also capable of predicting signal peptide-containing secretory proteins where only the mature part of the protein has been annotated or cases where the signal peptide remains uncleaved.

TargetP is a software tool for predicting the subcellular location of proteins by identifying the presence of N-terminal presequences, such as chloroplast transit peptide (cTP), mitochondrial targeting peptide (mTP), or secretory pathway signal peptide (SP).

Proteins which are secreted via the unconventional secretion pathway do not use the classical ER-Golgi pathway. Rather, these proteins are secreted through the unconventional secretion pathway which includes various mechanisms. Following endoplasmic reticulum (eR) translocation, signal-peptide-containing proteins are packaged into coat protein complex II (cOPII)-coated vesicles that fuse directly with the plasma membrane (mechanism 1). Alternatively, they can fuse with an endosomal or lysosomal compartment (such as late endosomes) that, in turn, fuses with the plasma membrane (mechanism 2). Proteins can also be packaged into non-cOPII-coated vesicles that can fuse directly with the plasma membrane (mechanism 3) or can be targeted to the Golgi apparatus (mechanism 4) before reaching the plasma membrane (see Nickel and Rabouille (2009), Nat Rev Mol Cell Biol. 10:148-155). Without being bound by theory, any one of the aforementioned mechansism 1-4 or all of them is/are envisaged to be used by the fungal host cell of the invention when secreting a protein via the unconventional secretion pathway.

It is envisaged that a polypeptide which is preferably secreted via the unconventional secretion pathway by a fungal host cell is preferably determined by an in silico analysis, in particular by the absence of a (secretion) signal peptide sequence (SignalP) and/or by the prediction that a protein is extracellularly located (Protocomp) and/or is predicted to be secreted via the unconventional secretion pathway (SecretomeP). Determination of a (secretion) signal sequence is preferably done as described herein above.

Once a protein is identified to be a candidate for secretion via the unconventional secretion pathway, a functional test can be preferably made. Accordingly, for example, Ustilago maydis cells can be used and the candidate protein can be fused in frame with ß-glucuronidase (GUS). In case the candidate protein is indeed secreted via the unconventional secretion pathway, GUS is not modified in the eR/Golgi. However, in case the candidate protein is secreted via the conventional pathway, GUS is modified in the eR/Golgi, thereby loosing some of its activity (Iturriaga et al. (1989), The Plant Cell 1 (3), 381-390).

In the present invention it was observed that in particular Cts1 (chitinase 1) from Ustilago maydis is secreted predominantly via the unconventional secretion pathway. In particular, it was observed that cts1 mRNA is transported along microtubules, and, based on further observations; it is assumed that cts1 mRNA is translated during its transport and/or at the hyphal tip, where it is delivered. Accordingly, if it is preferred to secrete a protein via the unconventional secretion pathway, a nucleotide sequence (a) can be applied which encodes a polypeptide which has at least 60% identity to the amino acid sequence of the amino acid sequence shown in SEQ ID No:2. The degree of identity between two amino acid sequences is preferably determined as described herein.

Likewise, if it is preferred to secrete a protein via the unconventional secretion pathway, a nucleotide sequence (a) can be applied which has at least 60%, 70%, 80%, 90% or 95% identity to the nucleotide sequence shown in SEQ ID No:1 or a fragment thereof and which encodes a protein which is secreted via the unconventional secretion pathway. Alternatively, a nucleotide sequence (a) can be applied which hybridizes to the nucleotide sequence shown in SEQ ID No:1 or a fragment thereof and which encodes a protein which is secreted via the unconventional secretion pathway.

The term "percent sequence identity" or "identical" in the context of nucleic acid sequences refers to the residues in the two sequences which are the same when aligned for maximum correspondence. The length of sequence identity comparison may be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides. There are a number of different algorithms known in the art which can be used to measure nucleotide sequence identity. For instance, polynucleotide sequences can be compared using FASTA, Gap or Bestfit, which are programs in Wisconsin Package Version 10.0, Genetics Computer Group (GCG), Madison, Wisconsin. FASTA provides alignments and percent sequence identity of the regions of the best overlap between the query and search sequences (Pearson, 1990, herein incorporated by reference). For instance, percent sequence identity between nucleic acid sequences can be determined using FASTA with its default parameters (a word size of 6 and the NOPAM factor for the scoring matrix) or using Gap with its default parameters as provided in GCG Version 6.1.

The term "substantial homology" or "substantial similarity," when referring to a nucleic acid or fragment thereof, indicates that, when optimally aligned with appropriate nucleotide insertions or deletions with another nucleic acid (or its complementary strand), there is nucleotide sequence identity in at least about 50%, more preferably 60% of the nucleotide bases, usually at least about 70%, more usually at least about 80%, preferably at least about 90%, and more preferably at least about 95%, 96%, 97%, 98% or 99% of the nucleotide bases, as measured by any well-known algorithm of sequence identity, such as FASTA, BLAST or Gap, as discussed above. Alternatively, substantial homology or similarity exists when a nucleic acid or fragment thereof hybridizes to another nucleic acid, to a strand of another nucleic acid, or to the complementary strand thereof, under stringent hybridization conditions. "Stringent hybridization conditions"and"stringent wash conditions"

In the context of nucleic acid hybridization experiments depend upon a number of different physical parameters. Nucleic acid hybridization will be affected by such conditions as salt concentration, temperature, solvents, the base composition of the hybridizing species, length of the complementary regions, and the number of nucleotide base mismatches between the hybridizing nucleic acids, as will be readily appreciated by those skilled in the art. One having ordinary skill in the art knows how to vary these parameters to achieve a particular stringency of hybridization.

In general, "stringent hybridization" is performed at about 25 C below the thermal melting point (Tm) for the specific DNA hybrid under a particular set of conditions. "Stringent washing" is performed at temperatures about 5 C lower than the Tm for the specific DNA hybrid under a particular set of conditions. The Tm is the temperature at which 50% of the target sequence hybridizes to a perfectly matched probe. See Sambrook et al., supra, page 9.51, hereby incorporated by reference. For purposes herein, "high stringency conditions" are defined for solution phase hybridization as aqueous hybridization (i.e., free of formamide) in 6X SSC (where 20X SSC contains 3.0 M NaCl and 0.3 M sodium citrate), 1% SDS at 65 C for 8-12 hours, followed by two washes in 0.2X SSC, 0.1 % SDS at 65°C for 20 minutes. It will be appreciated by the skilled artisan that hybridization at 65°C will occur at different rates depending on a number of factors including the length and percent identity of the sequences which are hybridizing.

The term "mutated" when applied to nucleic acid sequences means that nucleotides in a nucleic acid sequence may be inserted, deleted or changed compared to a reference nucleic acid sequence. A single alteration may be made at a locus (a point mutation) or multiple nucleotides may be inserted, deleted or changed at a single locus. In addition, one or more alterations may be made at any number of loci within a nucleic acid sequence. A nucleic acid sequence may be mutated by any method known in the art including but not limited to mutagenesis techniques such as "error-prone PCR" (a process for performing PCR under conditions where the copying fidelity of the DNA polymerase is low, such that a high rate of point mutations is obtained along the entire length of the PCR product. See, e.g., Leung, D. W., et al., Technique, 1, pp. 11-15 (1989) and Caldwell, R. C. & Joyce G. F., PCR Methods Applic., 2, pp. 28-33 (1992)); and "oligonucleotide-directed mutagenesis" (a process which enables the generation of site-specific mutations in any cloned DNA segment of interest. See, e.g., Reidhaar-Olson, J. F. & Sauer, R. T., et al., Science, 241, pp. 53-57 (1988)).

As nucleotide sequence (a) cts1 from *Ustilago maydis* shown in SEQ ID No.1 or a fragment thereof which encodes a protein secreted via the unconventional pathway is particularly preferred. The nucleotide sequence shown in SEQ ID No.1 encodes the amino acid sequence shown in SEQ ID No.2. Either the full-length protein or a fragment thereof which is secreted via the unconventional secretion pathway can be preferably used in the context of the invention.

In another preferred aspect of the invention, the expression cassette further comprises one or more (i.e., two, three, four, five, six and more) further nucleotide sequence(s) (a) fused in frame to the 5'- and/or 3'-end of the coding region of the nucleotide sequence of (a) and/or (b). Without being bound by theory, it is assumed that this preferred embodiment may enhance or make secretion more efficiently.

Preferably, the expression cassette of the invention comprising nucleotide sequence (a), (b) and/or (c) further comprise(s) one or more (i.e., two, three, four, five, six and more) further nucleotide sequence(s) (d) fused to the 5'- and/or 3'-end of the nucleotide sequence (a), (b) and/or (c). Accordingly, nucleotide sequence (d) is present "between" nucleotide sequences (a), (b) and/or (c) in the order as referred to herein in (i) to (vi).

In a preferred embodiment, nucleotide sequence (d) is comprised in the nucleotide sequence (a), (b) and/or (c). As described herein in the context of modifying nucleotide sequence (a) and/or (b) such that nucleotide sequence (c) is comprised in these nucleotide sequence(s), the nucleotide sequene (a), (b) and/or (c) can also be modified such that nucleotide sequence (d) is comprised in the nucleotide sequence (a), (b) and/or (c).

Preferably, nucleotide sequence (d) comprises at least 3 nucleotides, e.g., 3, 6, 9, 12, 15, 18, 21, 24, 27, 30 or more nucleotides. In a preferred embodiment, nucleotide sequence (d) comprises one or more (i.e., two, three, four, five, six and more) restriction enzyme recognition sites. These restriction enzyme recognition sites may be in the form of a "multiple cloning site", abbreviated as "MCS" and also known as a "polylinker".

Nucleotide sequence(s) (d) is/are prefeably fused in frame with the nucleotide sequence of (a), (b) and/or (c). Accordingly, if nucleotide sequence (d) is fused in frame with the nucleotide sequence of (a), (b) and/or (c), said nucleotide sequence (d) encodes a hereologous polypeptide.
Preferably, said heterologous polypeptide is a linker, tag and/or cleavage site for a protease.

A tag may be used to allow identification and/or purification of the protein of interest Examples of affinity tags that may be used in accordance with the invention include, but are not limited to, HAT, FLAG, c-myc, hemagglutinin antigen, His (e.g., 6xHis) tags, flag-tag, strep-tag, strepll-tag, TAP-tag, chitin binding domain (CBD), maltose-binding protein, immunoglobulin A (IgA), His-6-tag, glutathione-S-transferase (GST) tag, intein and streptavidie binding protein (SBP) tag. It is also envisaged that said heterologous polypeptide could be a whole immunoglobulin or, preferably any Fc region of an antibody such as FclgG, FclgA, FclgM, FclgD or FclgE.

A linker can be a peptide bond or a stretch of amino acids comprising at least one amino acid residue which may be arranged between the components of the fusion proteins in any order. Such a linker may in some cases be useful, for example, to improve separate folding of the individual domains or to modulate the stability of the fusion protein. Moreover, such linker residues may contain signals for transport, protease recognition sequences or signals for secondary modification. The amino acid residues forming the linker may be structured or unstructured. Preferably, the linker may be as short as 1 amino acid residue or up to 2, 3, 4, 5, 10, 20 or 50 residues. In particular cases, the linker may even involve up to 100 or 150 residues.

A cleavage site for a protease may be one for a serine protease, threonine protease, cysteine protease, aspartate protease, metalloprotease and/or glutamic acid protease.

The expression cassette of the invention is preferably driven by an expression control sequence, i.e. its expression is controlled by an expression control sequence which is preferably either a a constitutively active or inducible expression control sequence (preferably a promoter) that is operatively linked with the expression cassette. The term "expression" as used herein means the transcription of an expression cassette to produce the corresponding mRNA and translation of this mRNA to produce the corresponding gene product, such as a polypeptide, or protein.
"Operatively linked"expression control sequences refers to a linkage in which the expression control sequence is contiguous with the expression cassette, as well as expression control sequences that act in trans or at a distance to control expression of the expression cassette.
The term "expression control sequence" as used herein refers to polynucleotide sequences which are necessary to affect the expression of the expression cassette o which they are operatively linked. Expression control sequences are sequences which control the transcription, post-transcriptional events and translation of nucleic acid sequences. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (e.g., ribosome binding sites); sequences that enhance protein stability; and when desired, sequences that enhance protein secretion.
The term "'control sequences" is intended to include, at a minimum, all components whose presence is essential for expression, and can also include additional components whose presence is advantageous, for example, leader sequences and fusion partner sequences.

A promoter sequence is preferably inserted upstream of the expression cassette and regulates its expression. Promoter sequences are non-coding regulatory sequences for transcription, usually located nearby the start of the coding sequence, which may be referred to as the gene promoter or the regulatory sequence. Put into a simplistic yet basically correct way, it is the interplay of the promoter with various specialized proteins called transcription factors that determine whether or not a given coding sequence may be transcribed and eventually translated into the actual protein encoded by the gene. It will be recognized by a person skilled in the art that any compatible promoter can be used for recombinant expression in fungal host cells. The promoter itself may be preceded by an upstream activating sequence, an enhancer sequence or combination thereof. These sequences are known in the art as being any DNA sequence exhibiting a strong transcriptional activity in a cell and being derived from a gene encoding an extracellular or intracellular protein. It will also be recognized by a person skilled in the art that termination and polyadenylation sequences may suitably be derived from the same sources as the promoter.

In case of the host cell being Ustilago maydis, a preferred promoter is the constitutive tef, otef promoter (Spellig et al. (1996), Mol Gen Genet 252:503-509), hsp70 promoter (Holden et al., EMBO J. 8:1927-1934.. A preferred inducible promoter is the nar1 promoter (Brachmann et al., (2001), Mol Microbiol. 42:1047-63) or the crg1 promoter (Bottin et al. (1996), Mol Gen Genet 253:342-352)

The expression cassette of the invention may further comprise a nucleotide sequence encoding a marker protein. Preferably, said marker protein resistance against an antibiotic or anti-metabolite.
A marker protein, in accordance with the invention, means a protein which provides the transformed cells with a selection advantage (e.g. growth advantage, resistance against an antibiotic) by expressing the corresponding gene product. Marker genes code, for example, for enzymes causing a resistance to particular antibiotics. As used herein, the term "marker gene" refers to a gene whose product confers a characteristic to the cell expressing the marker gene that allows it to be distinguished from cells that do not express the marker gene. In some embodiments, the marker gene allows screening and/or selection of cells. In some such embodiments, the marker gene is a "screenable marker" or a "selectable marker". Screening and/or selection may be accomplished based on the presence or absence of the marker. In some embodiments, the screenable or selectable marker confers resistance to an agent such as an antibiotic. In some embodiments, the screenable or selectable marker confers an ability that provides an advantage in a particular set of growth conditions over cells that do not express the screenable or selectable marker.
As described above, the selectable marker can be the expression product of a gene encoding a protein restoring prototrophy for an organic compound, also referred to as prototrophy restoring gene. In thfs case, the selectable marker introduced enables the cell to synthesize said compound by itself so that it is no longer or less dependent on the external supply of said compound with the medium. Accordingly, a prototrophy restoring gene as used in the present invention is a gene encoding an expression product, i.e. the selectable marker, which reduces or preferably abolishes the dependency of the host cell on external supply of an organic compound by facilitating its synthesis in the cell. Selection for cells expressing said prototrophy restoring gene is carried out by culturing said cells on/in medium not containing said compound. Only cells expressing said prototrophy restoring gene will grow. The expression product of said gene may be a constituent of a synthesis pathway and the product produced by said constituent may have to be further processed in order to obtain the organic compound otherwise externally supplied. Prototrophy restoring genes commonly applied to plant or fungal cells are e.g. those expressing proteins conferring arginine prototrophy, tryptophan prototrophy, uridine prototrophy or genes enabling for nitrate or sulphate utilization. If the selectable marker is the expression product of a prototrophy restoring gene, the selecting agent is the medium in which the cell is cultivated and which does not contain the respective organic compound. Responsiveness in that case is expressed e.g. in growth rates of the cell. Thus, the higher the expression of the selectable marker, the higher the growth rate of the cell in the absence of the respective compound.
For some prototrophy restoring genes, the amount of expression product sufficient to result in prototrophy is very low. Accordingly, it is more laborious to distinguish cells expressing said prototrophy restoring selectable marker at a low level from those that express it at a high level. In order to facilitate said distinction, such a prototrophy restoring gene can be co-introduced together with a nucleic acid encoding a reporter gene the detectability of which is proportional to its expression level. Accordingly, in this embodiment, the selectable marker according to the invention is composed of the auxotrophy gene and the reporter gene.

In case the fungal host cell is an Ustilago maydis cell, preferred marker genes encode a resistance gene against hygromycin, G418, phleomycin, nourseothricin and carboxin.

In a further aspect, the invention relates to a vector comprising the expression cassette described herein.
The term "vector" as used herein refers to a nucleic acid sequence, e.g., DNA derived from a plasmid, cosmid, virus, or synthesized by chemical or enzymatic means, into which the expression cassette may be inserted or cloned, where the nucleic which encode for the nucleotide sequences described herein. Preferably the vector is an expression vector. A typical expression vector contains a promoter element, which mediates the initiation of transcription of mRNA, the protein coding sequence, and signals required for the termination of transcription and polyadenylation of the transcript. The vector can contain one or more unique restriction sites for this purpose, and may be capable of autonomous replication in a fungal host cell or may be ectopically or homologously integrated. The vector may have a linear, circular, or supercoiled configuration and may be complexed with other vectors or other material for certain purposes. The components of a vector can contain but is not limited to a DNA molecule incorporating DNA; a sequence encoding an excision protein or another desired product; and regulatory elements for transcription, translation, RNA stability, and replication.
The vector may comprise a polylinker (multiple cloning site), i.e. a short segment of DNA that contains many restriction sites, a standard feature on many plasmids used for molecular cloning. Multiple cloning sites typically contain more than 5, 10, 15, 20, 25, or more than 25 restrictions sites. Restriction sites within an MCS are typically unique (i.e., they occur only once within that particular plasmid). MCSs are commonly used during procedures involving molecular cloning or subcloning.
The expression cassette is inserted into the expression vector as a DNA construct. This DNA construct can be recombinantly made from a synthetic DNA molecule, a genomic DNA molecule, a cDNA molecule or a combination thereof. The DNA construct is preferably made by ligating the different fragments to one another according to standard techniques known in the art.
The gene coding for the protein of interest may be part of the expression vector. Preferably, the expression vector is a DNA vector. The vector conveniently comprises sequences that facilitate the proper expression of the expression cassette of the invention. These sequences typically comprise promoter sequences, transcription initiation sites, transcription termination sites, and polyadenylation functions as described herein. Additionally, the vector system may comprise a DNA sequence coding for a selection marker as described herein. Preferably, this selection marker is capable of being incorporated in the genome of the host organism upon transformation, and was not expressed functionally by the host prior to transformation. Transformed host cells can then be selected and isolated from untransformed cells on the basis of the incorporated selection marker.
Hence, according to one embodiment of the present invention the expression vector comprises a predefined restriction site, which can be used for linearization of the vector nucleic acid prior to transfection. Intelligent placement of said linearization restriction site is important, because said restriction site determines where the vector nucleic acid is opened/linearized and thus determines the order/arrangement of the expression cassettes when the construct is integrated into the genome of the fungal host cell. Vectors used for expressing the expression cassette including the nucleotide sequence coding for the protein of interest usually contain transcriptional control elements suitable to drive transcription such as e.g. promoters, enhancers, polyadenylation signals, transcription pausing or termination signals as elements of an expression cassette. For proper expression of the polypeptides, suitable translational control elements are preferably included in the vector, such as e.g. 5' untranslated regions leading to 5' cap structures suitable for recruiting ribosomes and stop codons to terminate the translation process. In particular, the nucleotide sequence serving as the selectable marker genes as well as the nucleotide sequence encoding the protein of interest can be transcribed under the control of transcription elements present in appropriate promoters. The resultant transcripts of the selectable marker genes and that of the protein of interest harbour functional translation elements that facilitate substantial levels of protein expression (i.e. translation) and proper translation termination.
According to one embodiment, the expression cassette(s) for expressing the polypeptide(s) of interest comprise(s) a stronger promoter and/or enhancer than the expression cassettes for expressing the selectable markers. This arrangement has the effect that more transcript for the polypeptide of interest is generated than for the selection markers. It is advantageous that the production of the polypeptide of interest which is secreted is dominant over the production of the selection markers, since the individual cell capacity for producing heterologous proteins is not unlimited and should thus be focused to the polypeptide of interest.

Furthermore, the expression cassettes may comprise an appropriate transcription termination site. This, as continued transcription from an upstream promoter through a second transcription unit may inhibit the function of the downstream promoter, a phenomenon known as promoter occlusion or transcriptional interference. This event has been described in both prokaryotes and eukaryotes. The proper placement of transcriptional termination signals between two transcription units can prevent promoter occlusion. Transcription termination sites are well characterized and their incorporation in expression vectors has been shown to have multiple beneficial effects on gene expression.
Most eukaryotic nascent mRNAs possess a poly A tail at their 3' end which is added during a complex process that involves cleavage of the primary transcript and a coupled polyadenylation reaction. The polyA tail is advantageous for mRNA stability and transferability. Hence, the expression cassettes of the vector according to the present invention usually comprise a polyadenylation site.
The expression cassettes may comprise an enhancer (see above) and/or an intron. According to one embodiment, the expression cassette(s) for expressing the polypeptide of interest comprise an intron. Usually, introns are placed at the 5' end of the open reading frame. Accordingly, an intron may be comprised in the expression cassette(s) for expressing the polypeptide(s) of interest in order to increase the expression rate. Said intron may be located between the promoter and or promoter/enhancer element(s) and the 5' end of the open reading frame of the polypeptide to be expressed. Several suitable introns are known in the state of the art that can be used in conjunction with the present invention
One type of vector is a "plasmid", which refers to a circular double stranded DNA loop into which additional DNA segments may be ligated. Other vectors include cosmids, bacterial artificial chromosomes (BAC) and yeast artificial chromosomes (YAC). Another type of vector is a viral vector, wherein additional DNA segments may be ligated into the viral genome (discussed in more detail below). Certain vectors are capable of autonomous replication in a host cell into which they are introduced (e. g., vectors having an origin of replication which functions in the host cell). Other vectors can be integrated into the genome of a fungal host cell upon introduction into the host cell, and are thereby replicated along with the host genome. Moreover, certain preferred vectors are capable of directing the expression of the expression cassette to which they are operatively linked. Such vectors are referred to herein as "recombinant expression vectors" (or simply,"expression vectors").

In a further aspect, the invention relates to a (recombinant) fungal host cell (including fungi and yeasts) comprising the expression cassette or the vector described herein. Preferably, the fungal host cell is capable of filamentous growth, preferably in liquid medium. Particularly preferred, the fungal host cell is *Ustilago maydis.*
The term"recombinant host cell" (or simply"host cell"), as used herein, is intended to refer to a fungal cell into which a nucleic acid comprising an expression cassette or vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated fungal cell, preferably grown in culture
The term "introducing a nucleic acid" refers to the application of a nucleic acid to fungal cells and its subsequent uptake and incorporation into the genetic information of said cells, in particular in the nucleus.
In general, the genetic alteration of a fungal cell resulting from the introduction/uptake and expression of foreign genetic material is termed "transformation" Yeasts and fungi may be transformed or by commonly known methods. By protoplast transformation, fungal cells can be converted to protoplasts by removing their cell wall, and can then be soaked in a solution containing DNA and transformed to become genetically modofied. The terms "genetically modified" and "transgenic" are used herein interchangeably. A transgenic or genetically modified fungal cell is one that has a genetic background which is at least partially due to manipulation by the hand of man through the use of genetic engineering. For example, the term "transgenic cell", as used herein, refers to a cell whose DNA contains an exogenous nucleic acid not originally present in the non-transgenic cell. A transgenic cell may be derived or regenerated from a transformed cell or derived from a transgenic cell.

A further aspect of the invention relates to a method for the production of the fungal host cell, siad method comprising transforming a fungal cell with the expression cassette or the vector described herein.

Likewise, the expression cassette or the vector described herein can be used for the production of a recombinant fungal host cell.

For example, the expression cassette or vector of the invention may either be integrated into the genome (ectopically or homologously) of the fungal cell or it may be maintained in some form extrachromosomally. Autonomously replicating sequences that can be used for the generation of free replicating vectors are, for example, known in Ustilago maydis (Tsukuda et al. (1988), Mol Cell Biol 8: 3703-3709).

In a yet further aspect, the invention concerns a method for the production of a polypeptide of interest comprising
(a) culturing the fungal host cell described herein to allow expression of said polypeptide;
(b) harvesting said polypeptide from the culture medium or fungal host cells such as from the cell wall as described herein.

Likewise, the fungal host cell or vector described herein can be used for the production of a polypeptide of interest.

The invention alse features a kit (expression system) comprising the expression cassette, the vector and/or the fungal host cell described herein, and optionally means for transforming a fungal host cell, fungal host cells as such, culture medium and/or an antibiotic or anti-metabolite for selecting and/or growing transformed fungal host cells.
In some embodiments, kits further comprise buffers for carrying out reactions and/or reagents for transforming cells with vectors.

The invention may also be charcterized by the following items:
1. Expression cassette comprising:
   (a) a first nucleotide sequence encoding a polypeptide which is secreted from a fungal host cell;
   (b) a second nucleotide sequence encoding a polypeptide of interest; and
   (c) one or more third nucleotide sequences which can be bound by a polypeptide comprising at least one sequence specific RNA binding domain,
      wherein the order (5'→ 3') of said first (a), second (b) and third (c) nucleotide sequence(s) is as follows:
      (i) (a), (b) and (c);
      (ii) (a), (c) and (b),
      (iii) (b), (a) and (c),
      (iv) (b), (c) and (a),
      (v) (c), (a) and (b), or
      (vi) (c), (b) and (a),
      wherein the nucleotide sequence of (a) and (b) or (b) and (a) are fused in frame.
2. The expression cassette of item 1 further comprising one or more further nucleotide sequence(s) (c) fused to the 5'- and/or 3'-end of the nucleotide sequence (a) and/or (b).
3. The expression cassette of item 1 further comprising one or more further nucleotide sequence(s) (a) fused in frame to the 5'- and/or 3'-end of the coding region of the nucleotide sequence of (a) and/or (b).
4. The expression cassette of any one of the preceding items, wherein the nucleotide sequences (a), (b) and (c) as referred to in (i) to (vi) are fused in frame.
5. The expression cassette of any one of the preceding items, wherein the nucleotide sequence(s) (c) is/are comprised in the nucleotide sequence (a) and/or (b).
6. The expression cassette of any one of the preceding items, wherein the nucleotide sequence (a), (b) and/or (c) comprise(s) one or more further nucleotide sequence(s) (d) fused to the 5'- and/or 3'-end of the nucleotide sequence (a), (b) and/or (c).
7. The expression cassette item 6, wherein the nucleotide sequence(s) (d) is/are comprised in the nucleotide sequence (a), (b) and/or (c).
8. The expression cassette of item 6 or 7, wherein said nucleotide sequence (d) comprises at least 3 nucleotides.
9. The expression cassette of item 6, 7 or 8, wherein said nucleotide sequence (d) comprises one or more restriction enzyme recognition sites.
10. The expression cassette of item 9, wherein the nucleotide sequence(s) (d) is/are fused in frame with the nucleotide sequence of (a), (b) and/or (c).
11. The expression cassette of any one of items 6-10, wherein said nucleotide sequence (d) encodes a hereologous polypeptide.
12. The expression cassette of item 11, wherein said heterologous polypeptide is a linker, tag and/or cleavage site for a protease.
13. The expression cassette of any one of the preceding items, wherein a constitutively active or inducible expression control sequence is operatively linked with the expression cassette.
14. The expression cassette of any one of the preceding items, wherein said first nucleotide sequence encodes a polypeptide which is secreted via conventional and/or unconventional protein secretion.
15. The expression cassette of any one of the preceding items, wherein said first nucleotide sequence encodes a polypeptide which has at least 60% identity to the amino acid sequence of the amino acid sequence shown in SEQ ID No:2
16. The expression cassette of any one of the preceding items, wherein said second nucleotide sequence encodes a polypeptide selected from the group consisting of enzymes including biocatalysts, receptors, receptor ligands such as competitors and scavenger receptors, antibodies, therapeutic proteins such as interferons, BMPs, GDF proteins, fibroblast growth factors, peptides such as protein inhibitors, membrane proteins, membrane-associated proteins, peptide/protein hormones, cytokines, peptidic toxins and peptidic antitoxins.
17. The expression cassette of any one of the preceding items, wherein said third nucleotide sequence comprises one or more (C/A)(C/A)(C/A) repeats, preferably CAA and/or CA, more preferably CA repeats.
18. The expression cassette of any one of the preceding items, wherein said third nucleotide sequence comprises the nucleotide sequence shown in SEQ ID No:3
19. The expression cassette of any one of the preceding items, wherein said at least one sequence specific RNA binding domain is an RNA recognition motif (RRM), which preferably comprises the amino acid sequence shown in SEQ ID No:4 (RRM motif).
20. The expression cassette of any one of the preceding items, wherein said first nucleotide sequence comprises the nucleotide sequence shown in SEQ ID No.1 (Cts1) or a fragment thereof encoding a polypeptide which allows secretion, preferably is secreted via unconventional secretion.
21. The expression cassette of any one of the preceding items further comprising a nucleotide sequence encoding a selection marker.
22. The expression cassette of item 21, wherein said selection marker confers resistance against an antibiotic or anti-metabolite.
23. A vector comprising the expression cassette of any one of the preceding items.
24. A fungal host cell comprising the expression cassette of any one of items 1-22 or the vector of item 23.
25. The fungal host cell of item 24 which is capable of filamentous growth, preferably in liquid medium.
26. The fungal host cell of item 24 or 25 which is *Ustilago maydis.*
27. A method for the production of the fungal host cell of item 24, 25 or 26 comprising transforming a fungal cell with the expression cassette of any one of items 1-22 or the vector of item 23.
28. A method for the production of a polypeptide comprising
   (a) culturing the fungal host cell of item 24, 25 or 26 to allow expression of said polypeptide;
   (b) harvesting said polypeptide from the culture medium or fungal host cells.
29. Use of the expression cassette of any one of items 1-22 or the vector of item 23 or the production of a recombinant fungal host cell.
30. Use of the expression cassette of any one of items 1-22 or fungal host cell of item 24, 25 or 26 for the production of a polypeptide.
31. A kit (expression system) comprising the expression cassette of any one of items 1-22, the vector of item 23 and/or the fungal host cell of item 24, 25 or 26, and optionally means for transforming a fungal host cell, fungal host cells, culture medium and/or an antibiotic for selecting and/or growing transformed fungal host cells.

The Figures show:

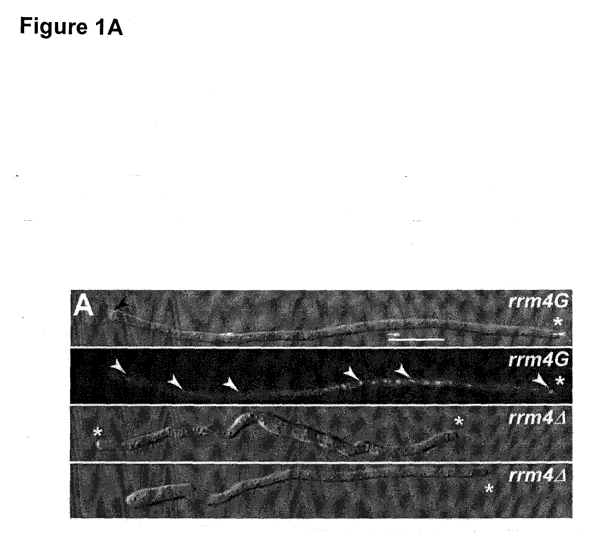

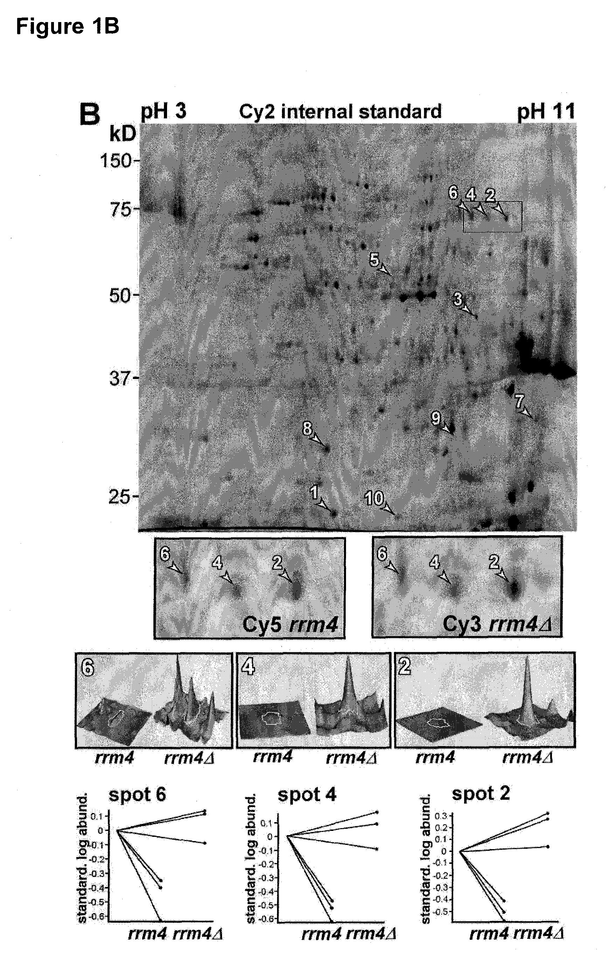

### Figure 1: 2D DIGE analysis identified 10 protein variants with altered amounts in the absence of Rrm4

*(A) rrm4Δ* strains are disturbed in filamentous growth. DIC images of AB33rrm4G and AB33rrm4Δ filaments 8 hours after induction are shown. These strains expressed Rrm4 fused to Gfp or carried a deletion in rrm4, respectively. Black or white arrowheads indicate retraction septa or Rrm4-containing particles, respectively. Growth cones are marked with asterisks (size bar = 10 µm). (B) Cy2-specific image of a representative 2D DIGE gel separating membrane-associated proteins derived of filament from AB33 and AB33rrm4Δ (size marker on the left). Ten protein variants exhibiting at least 2.5fold differences in protein amounts are indicated by numbered arrowheads. A region of the same gel is enlarged below, which shows Cy5 or Cy3-labelled proteins (left or right, respectively) covering spot 2, 4, and 6. 3D images document morphology of spots 2, 4, and 6. Spot boundaries defined by DeCyder software (GE Healthcare) are indicated as white lines. The bottom panels show scattered blot representations of the log abundance related to the normalised standard. Results of the three biological replicates analysed with three independent 2D DIGE gels are shown.

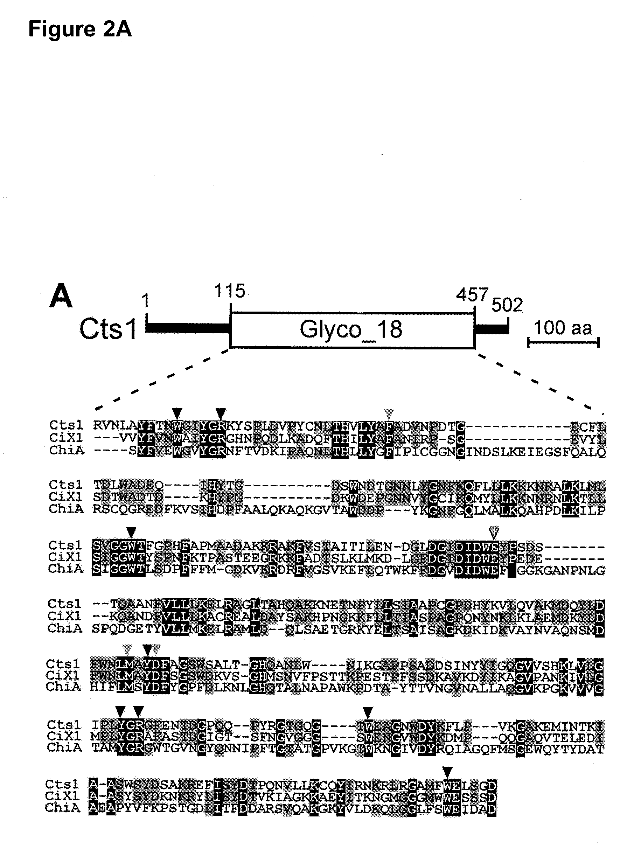

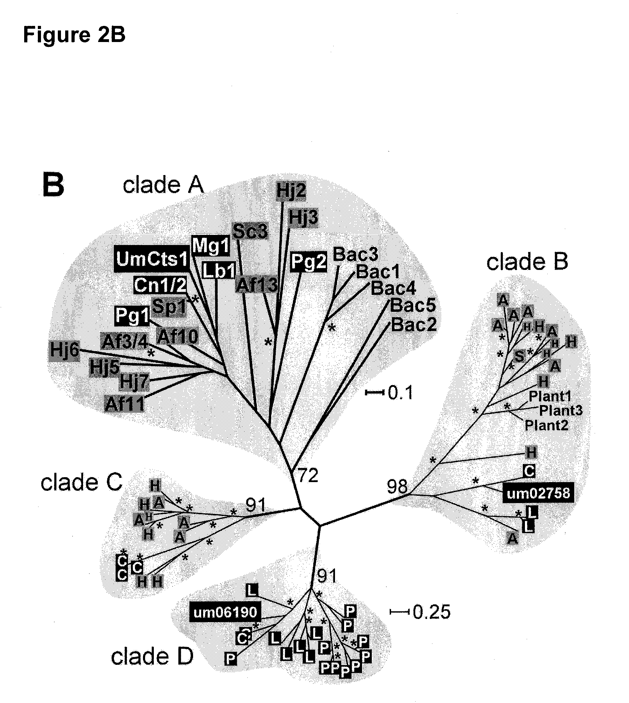

### Figure 2: um10419 encodes a bacterial-type chitinase of glycoside hydrolase family 18

(A) Upper part, schematic drawing of Cts1 containing a Glyco_18 domain (SMART) known from members of the glycoside hydrolase family 18. Lower part, the sequence of the Cts1 Glyco_18 domain is compared to sequences from fungal and bacterial chitinases CiX1 (*Coccidioides. immitis,* Hollis, T., et al. (2000), Protein Sci. 9:544-551) and ChiA (from *S*. *marcescens,* Perrakis et al. (1994), Structure:1169-1180), respectively. Identical amino acids in all three sequences or in two out of three sequences are shaded in black or grey, respectively. Arrowheads indicated conserved amino acids, which according to structural data form the substrate binding pocket or the active site of CiX1 (black) and ChiA (grey). (B) Phylogenetic tree of 78 enzymes belonging to the glycoside hydrolase 18 family. Representatives from bacteria (Bac1/2, S. marcescens; Bac3, Vibrio fisheri; Bac4/5 V. harveyi), higher plants (Plant1/2, *Nicotiana tabacum;* Plant 3, *Arabidopsis thaliana*) as well as fungi (ascomycetes and basidiomycetes are shaded grey and black, respectively) are shown. Two different scales are used. Maximum likelihood bootstrap values (1000 replicates) are given for the main branches (values above 90% are given as asterisks).

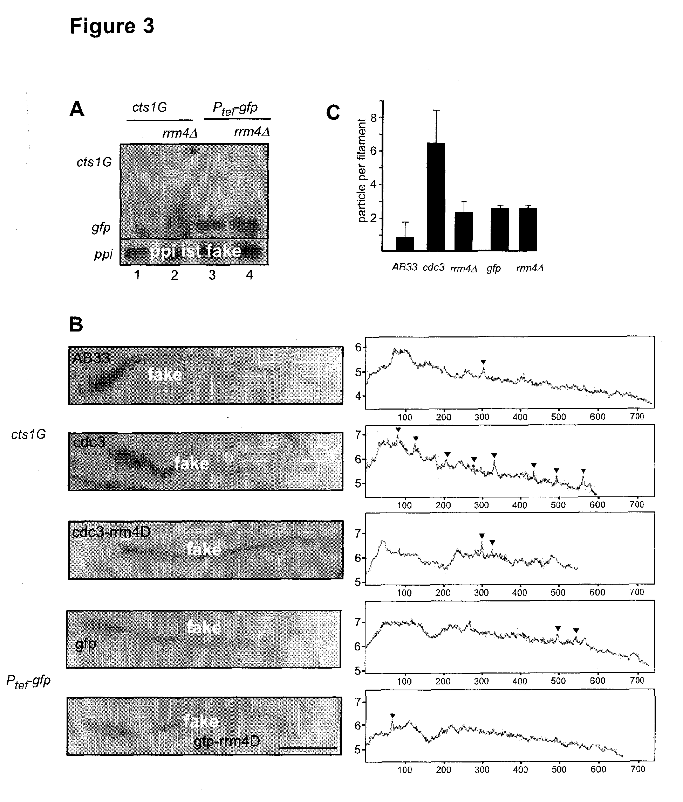

### Figure 3: cts1G mRNA preferentially accumulates in Rrm4-dependent particles

(A) Northern analysis comparing cts1G and gfp mRNA amounts (indicated on the left). The peptidy-prolyl cis-trans isomerase served as loading control. (B) FISH analysis of fixed AB33 filaments and derivatives (alleles given on the left). Inverted fluorescence images (left) including fluorescence intensity graphs (right) are documented. Relative fluorescence signals were plotted against the longitudinal axis of filaments (relative distance, rear pole was set to 0). Detection of peaks (filled arrowheads) was performed using PIA. (C) Bar diagram of mean particle numbers determined by PIA analysis (alleles labelled at the bottom; error bars, s.e.m., n = 3).

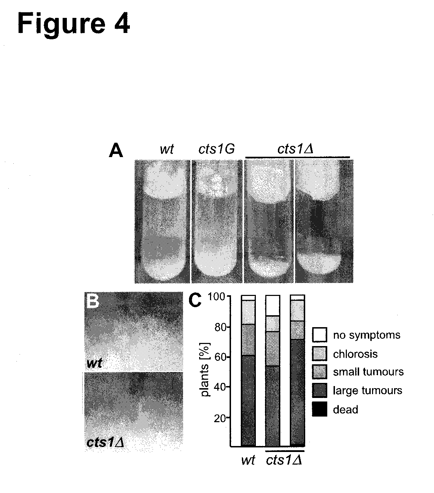

### Figure 4: cts1Δ filaments aggregate in liquid culture

(A) AB33 derivatives were grown for 16 hours in liquid minimal medium. (B) Edge of colonies (AB33 and AB33ctsΔ) incubated under filament inducing conditions on minimal medium plates for 24 hours. (C) Results of plant infection experiments with solopathogenic strains SG200 (Bölker, et al. (1995), Can. J. Bot. 73:320-325) and SG200cts1Δ. The percentage of plants with typical disease symptoms is given.

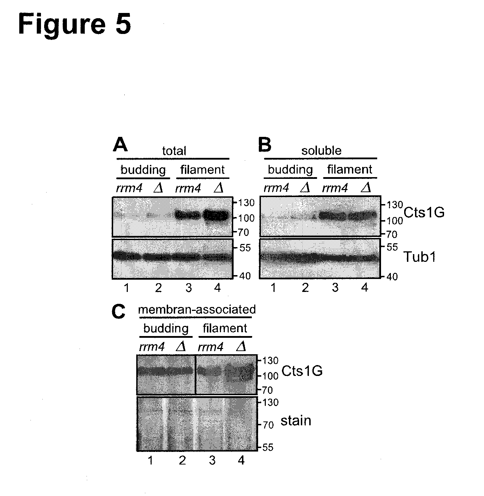

### Figure 5: Amount of Cts1G is increased during filamentation

Western analysis comparing budding cells and 8 hour old filaments of strains AB33 and AB33rrm4Δ. Total protein fractions (A), soluble protein factions (B), or membrane-associated fractions (C) were investigated. Detected proteins given on the right (marker in kD). Detection of α-tubulin Tub1 (in A and B) or protein staining (C) served was loading control.

### Figure 6: Cts1G accumulates at growth cones

DIC and epifluorescence images of budding cells and filaments are shown (upper and lower part, respectively; genotypes are indicated, size bar = 10 µm). Black and white arrowheads indicate retractions septa and specific accumulations of Cts1G at the poles, respectively.

### Figure 7: Secretion of Cts1 is impaired in rrm4Δ and kin1Δ strains

*A*) Bar diagram comparing endochitinolytic activity (relative fluorescence units) in budding cells and filaments (genotype is given below;). (B) Bar diagram comparing endochitinolytic activity (relative fluorescence units) in filaments of AB33 and AB33rrm4Δ in the presence of increasing concentrations of digitonin (note that fluorescence was measured with optimal gain settings, see Experimental procedures. Thus, RFUs cannot be directly compared between bar diagrams) (C) Bar diagram comparing endochitinolytic activity (relative fluorescence units) in filaments of different AB33 derivatives (genotypes are given). Strains were either untreated, treated with solvent control, or treated with benomyl. (D) Bar diagram as in C, but strains were treated in addition with digitonin for membrane permeabilisation as indicated below.

The following Examples illustrate the invention, but are not to be construed as limiting the scope of the invention.

### MATERIALS AND METHODS

### Strains and growth conditions

*E. coli* K-12 derivates DH5α (Bethesda Research Laboratories) and Top10 (Invitrogen) were used for cloning purposes. Growth conditions for *U. maydis* strains and source of antibiotics were described previously (Brachmann, et al. (2004), Mol. Gen. Genom. 272: 216-226). U. *maydis* strains were constructed by transformation of progenitor strains with linearised plasmids. Homologous integration events at the *ip, kin1* or *rrm4* locus were verified by Southern blot analysis (Brachmann, et al. (2004), Mol. Gen. Genom. 272: 216-226). For each experiment two independent transformants were analysed. Filamentous growth of AB33 derivates was induced by shifting cells of an exponential growing culture (OD₆₀₀ = 0.4 - 0.5) from liquid complete medium to nitrate minimal medium (NM). Cells were incubated at 28°C shaking with 200 rpm. For filamentous growth on plates, cells were incubated at 20°C for 24 hours on NM medium, containing 10 g/l activated charcoal and 20 g/l Bacto™ agar.

### Plasmids and plasmid constructions

Standard molecular techniques were followed. Plasmids pCR2.1-Topo (Invitrogen) and pBluescriptSKII (Stratagene) were used as cloning vehicles. Plasmid pRrm4Δ-HygR (pUMa495) was derived following published strategies to generate gene replacement mutants in *U. maydis* (Brachmann, et al. (2004), Mol. Gen. Genom. 272: 216-226). 0.9 kb upstream and 1.9 kb downstream flanking sequences of the ORF region were amplified with primer combinations MF220/MF803 and MF804/MF805, respectively. Genomic DNA of wild-type strain UM521 (*a1b1*) was used as template. Primers MF803 and MF804 introduced Sfil(u) and Sfil(d) recognition sites at the 3' end of the upstream flanking region and the 5' end of the downstream flanking region, respectively. PCR products were cleaved with Sfil and ligated in the presence of a compatible 1.8 kb Sfil(u)/Sfil(d) fragment (pUMa194) containing the hygromycin resistance cassette. The ligation product was cloned into pCR2.1-Topo. This plasmid was used to replace a 2376 bp region starting at nucleotide position -19 of the *rrm4* ORF (numbering relative to the translational start) by homologous integration of the hygromycin resistance cassette. Following the same strategy, plasmid pKin1Δ-HygR (pUMa542) was generated using Sfil-cleaved PCR products that were amplified using primer combinations MF787/MF788 (1 kb upstream flanking region) and MF789/MF790 (1.5 kb downstream flanking region). MF788 and MF789 introduced Sfil (u) and Sfil (d) recognition sites at the 3' end of the upstream flank and the 5' end of the downstream flank, respectively. By homologous integration a 3036 bp region starting at nucleotide position 6 of the *kin1* ORF is replaced by the hygromycin resistance cassette.
All constructions were confirmed by sequencing and all plasmid sequences are available upon request.

### Western Blot analysis

Whole cell extracts for determination of Rrm4G derivatives in Western blot analysis were prepared from 10 to 20 ml culture (OD₆₀₀ = 0.5). Cells were resuspended in 200 µl lysis buffer (100 mM sodium phosphate buffer, pH8; 10 mM Tris/HCl, pH8; 8 M urea; 1 mM PMSF; 0.5 mM benzamidine; 2x complete protease inhibitor cocktail [Roche]) and destroyed in the presence of glass beads in a pebble mill (Retsch, Germany; shaking for 7.5 minutes 30 times/second). After centrifugation (50 000g for 5 minutes at RT) protein concentration of the supernatant was determined by Bradford assay (Biorad) and 20 µg protein was loaded on SDS-PAGE and transferred to a PVDF membrane. Rrm4G derivatives were detected using α-Gfp antibody (Roche; mixture of two mouse monoclonal antibodies directed against Gfp) and α-mouse IgG HRP conjugate (H+L; Promega) as primary and secondary antibodies, respectively. HRP activity was detected using the ECL plus Western Blotting Detection System (Amersham Bioscience).

### DIGE analysis

DIGE analysis was done in accordance with the manufacturer's instructions.

### FISH analysis

FISH analysis was done in accordance with the procedure described in König et al. (2009), loc. cit..

### Inhibitor studies

1 ml of cell suspension was incubated in the presence of either 100 µM CCCP (Sigma-Aldrich), 50 µM latrunculin A (Sigma-Aldrich), or 20 µM benomyl (Sigma-Aldrich). Samples were incubated for 5 to 30 minutes at room temperature (RT) with agitation followed by microscopic analysis.

### Molecular phylogeny

Sequences of 78 chitinases were selected using the following criteria: firstly representatives of each subclass of basidiomycetes including saprophytes, mycorrhiza and parasites; secondly ascomycetes (whole repertoire of 2 well investigated species: A. *fumigatus* (Taib et al. (2005), Arch. Microbiol. 184:78-81) and *H. jecorina* (Seidl et al. (2005), FEBS J., 272: 5923-5939), S. *cerevisiae* and S. *pombe.* Thirdly, bacteria and plants.
Alignment of the selected sequences was performed with pcma v2.0 (Pei, et al. (2003), Bioinformatics, 19:427-428) with default settings resulting in an alignment of 3890 amino acids. The N- and C- terminal ends of the core alignment were defined as first and last conserved alignment block spanning five amino acids, respectively.
Highly variable regions (> 4 amino acids) encompassing from the C- and N-terminal ends until the first common motif spanning more than 4 amino acids were excluded revealing an alignment of 1028 positions including the catalytic centre of the protein. RAxML 7.0.4 (Stamatakis (2006), Bioinformatics, 22:2688-2690) was used to perform a full maximum likelihood analysis in which 1000 bootstrap replicates were conducted using a rapid algorithm (Stamatakis, et al. (2008), Syst. Biol., 57, 758-771) applying PROTCAT approximation under the BLOSUM62 amino acid model. The subsequent ML search for the best scoring ML tree starting from each 5th BS tree was conducted with the more accurate PROTGAMMA approximation under the same amino acid model.

### RESULTS

### 2D DIGE identifies ten significant differences comparing wild type and rrm4Δ strains

In order to investigate the role of Rrm4 during formation of infectious filaments we applied difference in gel electrophoresis (DIGE; Westermeier and Scheibe (2008), Methods Mol. Biol., 424, 73-85). As genetic background, we chose laboratory strain AB33 that is well-suited to study filamentation. Since this strain expresses an active bW2/bE1 heterodimeric transcription factor under control of the nitrate-inducible *nar1* promoter (Brachmann et al. (2001), Mol. Microbiol.:42, 1047-1063), b-dependent filament formation can be elicited by changing the nitrogen source of the medium (Fig. 1A). In pilot 2D PAGE experiments, we observed a number of differences when comparing the membrane-associated protein fraction of strains AB33 and AB33rrm4Δ Thus, these protein fractions of strains, which were grown for 8 hours under filament inducing conditions (three biological replicates), were subjected to DIGE analysis. Proteins of both strains were covalently labelled with CyDyes™ Cy5 or Cy3 (GE Healthcare) using the minimal labelling technique. As an internal standard all six protein samples were mixed and covalently labelled with Cy2 (Gade et al. (2003), J. Mol. Microbiol. Biotechnol., 5, 240-251; Westermeier and Scheibe (2008), loc. cit). Cy2, Cy3 and Cy5 labelled protein samples of biological replicates were co-separated in three independent gels (Fig. 1B). Applying a threshold of 2.5fold, 10 protein variants out of approximately 600 protein spots were identified (DeCyder™ software, GE healthcare). Average ratios ranged from 3 to 15fold and Student t test values from 0.01 to 7 * 10⁻⁴ (Fig. 1B; Table 1). Consistently, the same ten variants were identified in a fourth biological replicate performing dye swap experiments (AB33 and AB33rrm4Δ proteins labelled with Cy3 and Cy5, respectively; data not shown).

Seven protein variants could be identified by preparative 2D PAGE, labelling with *Deep Purple* (GE healthcare), and subsequent mass spectrometry analysis (Table I, for mass spectrometry data see Supplementary Table I). Spot 1 exhibiting 15fold increase in *rrm4Δ* filaments corresponded to ribosomal protein Rps19. Spot 3, 8, and 10 corresponded to the mitochondrial proteins Afg3, Atp4 and Nuo2, respectively (Table I). Three protein variants (spot 2, 4 and 6) with comparable molecular weight but differences in pl corresponded to um10419 encoding a bacterial-type endochitinase Cts1. The amount of all three versions is increased in *rrm4* Δ filaments indicating that various posttranslational modified variants accumulate in the absence of Rrm4. In summary, subcellular proteomics reveals that the amount of specific proteins implicated in cell wall degradation, translation, or mitochondrial functions are altered in the absence of Rrm4. In the remaining study we focussed on endochitinase Cts1.

### um10419 encodes the bacterial-type endochitinase Cts1 containing a highly conserved active site

Sequence analysis of um10419 using SMART (Letunicet al. (2004), Nucleic Acids Res., 32, D142-144) and MUMDB (http://mips.gsf.de/genre/proj/ustilago; Kämper et al., (2006), Nature, 444, 97-101) revealed that the gene contained a single 81 bp intron and encoded a protein of 502 amino acids (55 kDa; isoelectric point 8.2). The protein harboured a Glyco_18 domain characteristic for endochitinases that belong to the glycoside hydrolase family 18. Therefore, the protein was annotated as endochitinase Cts1. This was supported by a BLAST analysis exhibiting high sequence similarity to endochitinases from bacteria and fungi (Fig. 2A). Importantly, 13 amino acids are conserved in Cts1, which form the substrate binding pocket and active site in ChiA and CiX1 from Serratia marcescens and Coccidioides immitis, respectively (Hollis et al., Protein Sci., 9, 544-551; Perrakis, et al. (1994), Structure, 2, 1169-1180). We noticed that additional sequence motifs found in other endochitinases were missing such as N-terminal secretion signal, chitin-binding domain, cellulose-binding domain or GPI-anchor attachment motif (glycosylphosphatidylinositol; Dünkler et al. (2005), Fungal Genet. Biol., 42, 935-947; Seidl et al., (2005)FEBS J., 272, 5923-5939; Yamazaki et al. (2008), Fungal Genet. Biol., 45, 963-972).

Molecular phylogeny comparing a representative selection of 80 chitinase sequences from fungi, plants and bacteria (see Experimental procedures) revealed that the selected family 18 glycoside hydrolases group in four monophyletic clades. Members of clade A are recognized as class III or bacterial-type chitinases and members of clade B are class V or plant-type chitinases. Members of clade C belong to a group of high molecular weight chitinases that have been recently classified analysing chitinases of Hypocrea jecorina Hypocrea jecorina (Seidl et al. (2005), loc. cit.) whereas Clade D comprises basidiomycete-specific enzymes (Fig. 2B; Supplementary Fig. 1). This analysis demonstrates that Cts1 belongs to the bacterial-type endochitinases of clade A (Fig. 2B). In contrast to cts1, the two remaining genes um02758 and um06190 encode predicted endochitinases (MUMDB) that belong to clade B and D, respectively. Both contain an N-terminal secretion signal (predicted by Signal-P implemented in SMART). Thus, U. maydis contains three family 18 endochitinases that belong to separate clades, supporting very ancient gene duplication and non-redundant functions.

### cts1G mRNA accumulates preferentially in Rrm4-dependent mRNP particles

To address whether cts1 mRNA is a direct target of Rrm4 we compared the subcellular localisation of cts1G and gfp mRNA performing fluorescent in situ hybridisation (FISH, König et al., 2009). The later encoded the enhanced version of the green fluorescence protein (eGfp, Clontech) and served as heterologous control mRNA. For optimal comparison we used the same set of DNA oligonucleotides directed against gfp (König et al., 2009). Thus, we generated strain AB33cts1G expressing Cts1 C-terminally fused to Gfp using a Sfil-mediated homologous gene replacement system (Brachmann et al. (2004), Mol. Gen. Genom., 272, 216-226; see Experimental procedures). Filaments of the resulting strain did not exhibit the mutant cuts1Δ phenotype (see Fig. 4A), indicating that the fusion protein was fully functional. The control strain expressed gfp under control of the constitutively active promoter Ptef (AB33Ptef-gfp, Spellig et al. (1996), loc. cit). To investigate Rrm4 dependence of mRNA localisation we generated respective rrm4Δ strains.

Analysing this set of strains in Northern blot experiments revealed that deletion of rrm4 had no influence on the amount of cts1G or gfp mRNA (Fig. 3A, lane 1 and 3). Noteworthy, the amount of gfp control mRNA higher than cts1 G mRNA.

For FISH experiments, filaments were grown for 8 h under inducing conditions and fixed with formaldehyde. In AB33cts1G, hybridisation revealed a punctuated staining of cts1G mRNA in particles throughout the cytoplasm (Fig. 3B). These particles were drastically reduced in AB33cts1G/rrm4Δ and AB33 indicating that the cts1G mRNA particles are Rrm4 dependent and that probes are specific, respectively (Fig. 3B). Consistent with other mRNA targets of Rrm4, no subcellular accumulation or mRNA gradient, for example, at the poles of filaments were detected (König, (2009), EMBO J. 28:1855-1866).

To enable objective detection and statistical evaluation, punctuated staining was quantified as peaks in two-dimensional line scans derived from microscopic images using a specifically designed peak-identifying algorithm (PIA, Fig. 3B; see Experimental procedures; König et al. (2009), loc. cit.).

Analysing filaments revealed that particles were detected in AB33cts1G. These particles were Rrm4 dependent since deletion resulted in hardly any particles (Fig. 3A, B). In case of AB33Ptefgfp, the amount of particles was significantly lower. Also in this case the particles were Rrm4 dependent (Fig. 3A, B). These results were consistent with early observations showing that in principle all mRNAs are found in Rrm4-dependent particles. However, direct targets of Rrm4 are present with increased frequency in transport particles (König et al. (2009), loc. cit.).

In essence, although cts1G mRNA amount is lower than the control mRNA it is found significantly more often in Rrm4 particles supporting the conclusion that cts1G mRNA is a direct target of Rrm4-dependent mRNA transport.

### Loss of cts1 causes aggregation of filaments

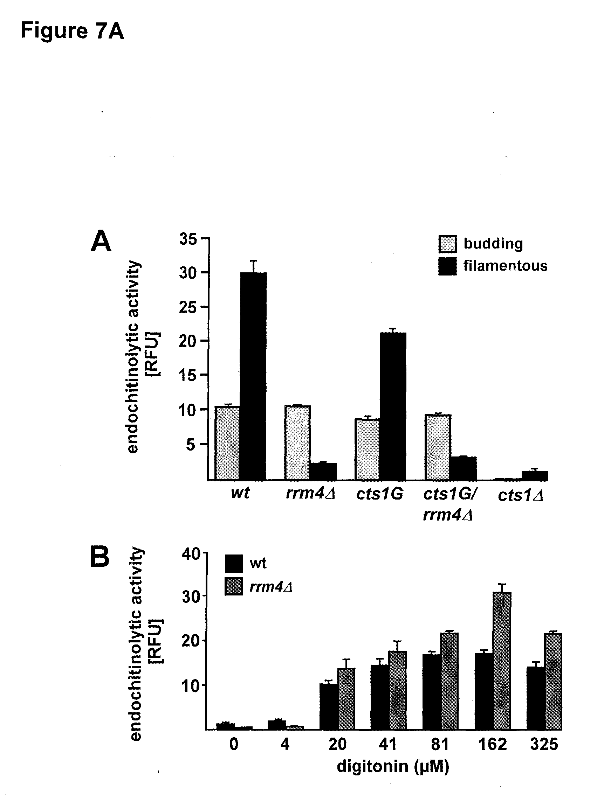

To investigate the role of Cts1 during filamentous growth we deleted the corresponding gene in AB33 using a Sfil-mediated homologous gene replacement system (Kämper (2004), Mol. Gen. Genom. 271:103-110). Measuring the chitinolytic activity of cts1Δ filaments using a substrate specific for endochitinases revealed that enzyme activity was drastically reduced confirming that Cts1 functions as endochitinase (Fig. 7A, see below).

During budding no phenotype of cts1Δ strains was observed indicating that the enzyme is not essential for separation of mother and daughter cells. However, cts1Δ filaments differed from wild type, since in liquid culture, mutant filaments tend to flocculate and stick to the glass surface (Fig. 4A). Microscopic analysis revealed that deletion filaments formed large aggregates, in which empty sections composed of cell wall remnants sticked together more pronounced than in wild type. However, filament morphology, insertion of retraction septa, as well as filament length was comparable to wild type (Fig. 4B). Testing different stress conditions such as cell wall, salt, and osmotic stress did not reveal differences in cts1Δ strains and wild type (growth on plates in the budding and filamentous form in the presence of 50 µM calcoflour and 2 M congo red, 2% hydrogenperoxide, 1 M sodiumchloride, and 1 M sorbitol). Thus, no major defects in cell wall function was observed in the absence of Cts1.

For plant infection experiments we deleted cts1 in the solopathogenic strain SG200, enabling plant infection experiments without prior mating (Bölker et al. (1995), Can. J. Bot. 73:320-325). Infection of corn seedlings revealed no difference between SG200 and SG200cts1Δ indicating that the chitinase was dispensable for infection (Fig 4C). In essence, loss of Cts1 led to aggregation during filamentous growth. However, this difference did not cause major alterations in plant penetration, proliferation and tumour induction.

### The amount of Cts1 is specifically increased in membrane-associated fractions of proteins

For analysis of the subcellular localisation of Cts1 we tested AB33cts1G and the corresponding rrm4 deletion strain in Western blot experiments. Comparing whole cell extracts of budding cells and filaments revealed that the amount of Cts1 G was drastically increased during filamentation (Fig. 5A, lane 1, 3). Comparing wild type and rrm4Δ strain showed that in the absence of Rrm4 the amount of Cts1 G was elevated in budding cells and filaments (Fig. 5A, lane 1 - 2 and 3 - 4; respectively). In soluble protein fractions, Cts1 G was hardly increased in wild type and rrm4Δ strain (Fig. 5B, lane1 - 2 and 3 - 4). The same holds true in membrane associated protein fractions of budding cells (Fig. 5C, lane 1 - 2). However, in filaments, the amount of Cts1 G was significantly higher in the rrm4Δ strain (Fig. 5C, lane 3 - 4). Noteworthy, this confirmed our DIGE data. In essence, Cts1 amount increases specifically during filamentous growth and loss of Rrm4 causes an increased abundance of the endochitinase Cts1 in the membrane-associated protein fraction of filaments.

### Cts1G accumulates at caps of growth cones and mislocalises in rrm4Δ strains

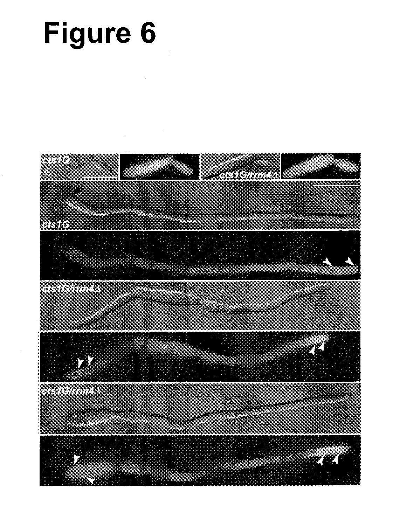

Fluorescence microscopy of the same set of strains revealed that in budding cells Cts1G was evenly distributed throughout the cytoplasm and that a faint signal was observed in the membrane (Fig. 6, top). Cts1 G did not accumulate at septa during budding consistent with the observation that there was no defect in budding (see above). In accordance with our Western blot analysis, the Cts1 G localisation was comparable in wild type and the rrm4Δ strain (Fig. 5A).

In filaments, Cts1G localises predominantly at membranes of growth cones (Fig. 6, bottom). No accumulation at retraction septa was observed. In bipolar filaments of the rrm4Δ strain, Cts1G accumulated at membrane caps of both growing poles. Interestingly, Cts1G accumulated even in unipolar filaments of rrm4Δ strains at both poles: at the membrane of growing tips as well as at the membrane of the initial cell (Fig. 6, bottom). This result is consistent with the increased accumulation of Cts1 G in protein fractions of membrane-associated proteins. In summary, Cts1 G accumulates at hyphal tips and mislocalises in the absence of Rrm4.

### rrm4Δ filaments are disturbed in secretion of Cts1G

In order to correlate subcellular localisation with enzyme activity, we determined endochitinolytic activity using a fluorigenic substrate that is specific for endochitinase activity (see Experimental procedures). In pilot experiments comparing supernatant and washed cells or filaments, we observed that most of the enzyme activity was associated with cells and filaments. This suggests that, extracellular endochitinase binds to the cell wall.

Measuring whole cells revealed that in budding cells deletion of rrm4 did not alter enzyme activity (Fig. 7A, light grey bars). This is consistent with our previous data (Fig. 5-6). Importantly, deletion of cts1 caused a drastic reduction in enzyme activity. Thus, Cts1 is the major endochitinase and potentially redundant enzymes play marginal roles (see phylogenetic analysis in Fig. 2B).

Measuring endochitinolytic activity in whole filaments revealed that hydrolytic activity was increased during filamentation. This observation is consistent with our Western blot analysis (Fig. 5A). Surprisingly, filaments carrying a deletion in rrm4 resulted in a drastically decreased activity, although determination of Cts1 G amount indicated a clear increase (Fig.1B, 5A). To solve this apparent discrepancy we measured endochitinolytic activity in the presence of increasing amounts of digitonin resulting in progressive membrane permeablisation. Comparing wild type and rrm4Δ strain revealed that in the absence of digitonin and at low concentrations endochitinase activity was elevated in wild type. At higher concentrations, overall endochitinolytic activity increased and activity was always higher in rrm4Δ strains. Thus, a substantial amount of active enzyme resides inside filaments and importantly, the secretion of Cts1 is drastically impaired in the absence of Rrm4.

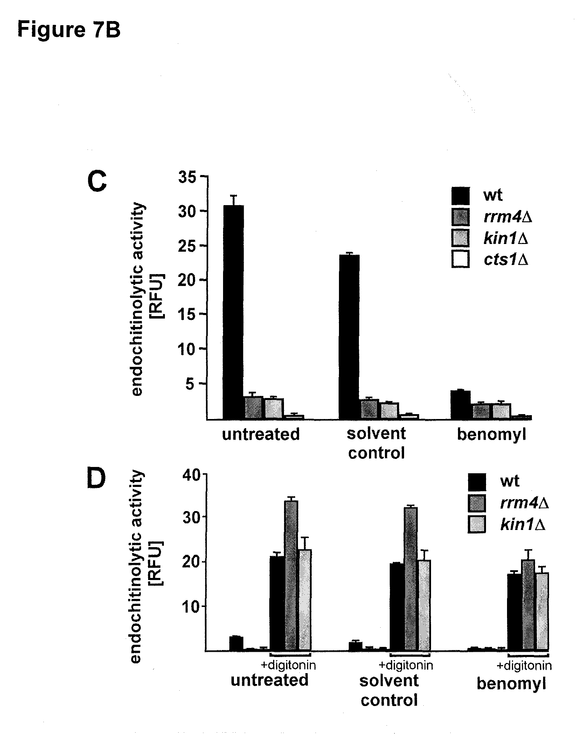

In order to test whether this impaired secretion was connected to the function of Rrm4 during microtubule-dependent mRNA transport, we measured endochitinolytic activity while interfering with the function of microtubules. To this end cells were either treated with the microtubule-inhibitor benomyl (Fuchs et al. (2005), Mol. Biol. Cell. 16:2746-2758) or activity was tested in an AB33kin1Δ carrying a mutation in the conventional kinesin kin1. Loss of this molecular motor resulted in accumulation of Rrm4-containing particles at hyphal tips (Becht et al. (2006), J. Cell Sci., 119:4964-4973). Measuring endochitinolytic activity in whole filaments revealed that Cts1 activity was drastically reduced in those filaments that were treated with benomyl for 4 hours. Consistently, Cts1 activity was low in kin1Δ strains (Fig. 7C). To verify that the drastic decrease of Cts1 activity in the presence of benomyl or in kin1Δ strains was due to impaired secretion of Cts1, benomyl-treated filaments and kin1Δ filaments were incubated in the prescence digitonin. Due to membrane permeabilisation endochitinolytic activity increased (Fig. 7D). Thus, microtubules, Kin1 and Rrm4 are essential for efficient secretion of Cts1 consistent with our hypothesis that microtubule-dependent mRNA transport mediated by Rrm4 supports secretion of Cts1 in infectious filaments.

**Table 1. Protein variants identified by DIGE**

| **Identifier** | **Relative fold difference^{a}** | **Student T test** | **Unique peptides detected** | **Sequence coverage %** | **Expectation value** | **um number^{b}** | **Gene^{b}** | **Predicted gene function^{b}** |
|---|---|---|---|---|---|---|---|---|
| 1 | 14.7 | 0.0005 | | | | um04662 | Rps1 | probable RPS19B- ribosomal protein S19 |
| 2 | 5.3 | 0.0019 | | | | um10419 | Cts1 | ch itinase |
| 3 | 4.5 | 0.0047 | | | | um00898 | Afg3 | probable AFG3 - protease of the SEC18/CDC48/PAS1 family of ATPases (AAA) |
| 4 | 4.1 | 0.0028 | | | | um10419 | Cts1 | ch itinase |
| 5 | 3.3 | 0.0047 | | | | n. i.^{c} | | |
| 6 | 3.2 | 0.01 | | | | um10419 | Cts1 | ch itinase |
| 7 | 3 | 0.0013 | | | | n. i.^{c} | | |
| 8 | - 3.1 | 0.0052 | | | | um10548 | Atp4 | probable H+-transporting two-sectorATPase chain b precursor, mitochondrial |
| 9 | - 3.6 | 0.0061 | | | | n. i.^{c} | | |
| 10 | -5.8 | 0.0007 | | | | um11495 | Nuo2 | related to nadh-ubiquinone oxidoreductase 21.3 kDa subunit |

| | | | | | | | | |
|---|---|---|---|---|---|---|---|---|
| ^{a} *rrm4* versus wildtype - ^{b} MUMDB, http://mips.gsf.de/genre/proj/ustilago/ (Kämper et al. (2006), loc. cit.) ^{c} not identified | | | | | | | | |

## Claims

1. Expression cassette comprising:
(a) a first nucleotide sequence encoding a polypeptide which is secreted from a fungal host cell;
(b) a second nucleotide sequence encoding a polypeptide of interest; and
(c) one or more third nucleotide sequences which can be bound by a polypeptide comprising at least one sequence specific RNA binding domain,
wherein the order (5'→ 3') of said first (a), second (b) and third (c) nucleotide sequence(s) is as follows:
(i) (a), (b) and (c);
(ii) (a), (c) and (b),
(iii) (b), (a) and (c),
(iv) (b), (c) and (a),
(v) (c), (a) and (b), or
(vi) (c), (b) and (a),
wherein the nucleotide sequence of (a) and (b) or (b) and (a) are fused in frame.

2. The expression cassette of claim 1, wherein the nucleotide sequences (a), (b) and (c) as referred to in (i) to (vi) are fused in frame.

3. The expression cassette of any one of the preceding claims, wherein the nucleotide sequence(s) (c) is/are comprised in the nucleotide sequence (a) and/or (b).

4. The expression cassette of any one of the preceding claims, wherein the nucleotide sequence (a), (b) and/or (c) comprise(s) one or more further nucleotide sequence(s) (d) fused to the 5'- and/or 3'-end of the nucleotide sequence (a), (b) and/or (c).

5. The expression cassette claim 4, wherein the nucleotide sequence(s) (d) is/are comprised in the nucleotide sequence (a), (b) and/or (c).

6. The expression cassette of claim 4 or 5, wherein the nucleotide sequence(s) (d) is/are fused in frame with the nucleotide sequence of (a), (b) and/or (c).

7. The expression cassette of any one of the preceding claims, wherein said first nucleotide sequence encodes a polypeptide which is secreted via conventional and/or unconventional protein secretion.

8. The expression cassette of any one of the preceding claims, wherein said first nucleotide sequence encodes a polypeptide which has at least 60% identity to the amino acid sequence of the amino acid sequence shown in SEQ ID No:2.

9. The expression cassette of any one of the preceding claims, wherein said third nucleotide sequence comprises one or more (C/A)(C/A)(C/A) repeats, preferably CAA and/or CA, more preferably CA repeats.

10. The expression cassette of any one of the preceding claims, wherein said third nucleotide sequence comprises the nucleotide sequence shown in SEQ ID No:3

11. A vector comprising the expression cassette of any one of the preceding claims.

12. A fungal host cell comprising the expression cassette of any one of claims 1-10 or the vector of claim 11.

13. The fungal host cell of claim 12 which is capable of filamentous growth, preferably in liquid medium.

14. The fungal host cell of claim 12 or 13 which is *Ustilago maydis.*

15. A method for the production of a polypeptide comprising
(a) culturing the fungal host cell of claim 12, 13 or 14 to allow expression of said polypeptide;
(b) harvesting said polypeptide from the culture medium or fungal host cells.


SEQUENCE LISTING

<110>  Heinrich-Heine-Universität Düsseldorf

<120>  Protein production in filamentous fungi

<130>  HHU13611EP

<160>  5

<170>  PatentIn version 3.5

<210>  1
<211>  1509
<212>  DNA
<213>  Ustilago maydis

<400>  1
atgtttggac gtcttaagca caggatgtct cgcgctcgac tagacgacga cggcaaaaag      60

tcatcgtcat ccgcttcatc gttgccacct tctccaacca aggcagcaac agcctctgct     120

gctggttcgg tccctcagac tccgactgct acggcacccg aagcttccac gccctcatcc     180

tcgacccaac ctgagtctcc cgtcgcttcg gcccccagca gcacttctcc tccgagtacg     240

acacctacca caccagcttc caacacaacc cctgcaagcg agatccaaaa caacatcgac     300

tcgcaaggtc acgatttcac caccaatggt gcggtcgtcc ctcgtgtcaa cctcgcctac     360

tttacaaact ggggtatcta cggtcgcaag tactctcctc tggacgttcc ttactgcaac     420

ctgacgcacg ttctctacgc tttcgccgac gtcaaccccg acaccggcga gtgcttcctc     480

accgatctct gggccgacga gcaaattcac tacactggcg actcctggaa cgatacgggt     540

aacaacctct acggcaactt taagcaattc cttttgctca agaagaagaa ccgtgcgctc     600

aagctcatgc tgtccgttgg tggatggaca tttggtccgc actttgcacc tatggcagcc     660

gacgccaaga agcgagccaa gtttgtctcg actgccatca cgattttgga gaacgacggg     720

ttggatggaa tcgacatcga ctgggagtat ccctcggaca gcacgcaagc tgccaacttt     780

gttctgctgc tcaaagagct tcgtgccggc ttgactgcac accaggccaa gaagaacgag     840

accaacccgt acctactgtc catcgctgcg ccctgtggac ctgaccatta caaggtgctc     900

caagtagcca agatggacca gtacctggat ttctggaacc tcatggcgta cgatttcgcc     960

ggttcgtggt ccgcactcac cggccaccaa gccaacctgt ggaacatcaa gggtgcaccg    1020

ccttcggccg acgattcgat caactactat attggccagg gcgtcgtttc gcacaagctc    1080

gttctcggaa tcccgctcta cggccgtggc ttcgaaaaca cggacggtcc acaacagccg    1140

tacaggggta cagggcaggg cacgtgggaa gccggcaact gggactacaa gtttttgcct    1200

gtcaaaggtg caaaggagat gatcaacacc aagatcgctg ctagttggag ttacgactca     1260

gccaagcgcg agttcattag ttacgacaca cctcagaacg tcttgttgaa gtgtcagtac     1320

atcaggaaca agaggttgag gggtgccatg ttctgggagc tgagtggtga cgcaaccaag     1380

agccagggtg gtgctgaaag gagtctcatc gccctcactg ccaagaacat gggcacactc     1440

gatgccactt tgaaccacat cagttaccct ttcagcaagt gggacaatgt caagaacggc     1500

ctcaagtaa                                                             1509

<210>  2
<211>  502
<212>  PRT
<213>  Ustilago maydis

<400>  2

Met Phe Gly Arg Leu Lys His Arg Met Ser Arg Ala Arg Leu Asp Asp
1               5                   10                  15

Asp Gly Lys Lys Ser Ser Ser Ser Ala Ser Ser Leu Pro Pro Ser Pro
            20                  25                  30

Thr Lys Ala Ala Thr Ala Ser Ala Ala Gly Ser Val Pro Gln Thr Pro
        35                  40                  45

Thr Ala Thr Ala Pro Glu Ala Ser Thr Pro Ser Ser Ser Thr Gln Pro
    50                  55                  60

Glu Ser Pro Val Ala Ser Ala Pro Ser Ser Thr Ser Pro Pro Ser Thr
65                  70                  75                  80

Thr Pro Thr Thr Pro Ala Ser Asn Thr Thr Pro Ala Ser Glu Ile Gln
                85                  90                  95

Asn Asn Ile Asp Ser Gln Gly His Asp Phe Thr Thr Asn Gly Ala Val
            100                 105                 110

Val Pro Arg Val Asn Leu Ala Tyr Phe Thr Asn Trp Gly Ile Tyr Gly
        115                 120                 125

Arg Lys Tyr Ser Pro Leu Asp Val Pro Tyr Cys Asn Leu Thr His Val
    130                 135                 140

Leu Tyr Ala Phe Ala Asp Val Asn Pro Asp Thr Gly Glu Cys Phe Leu
145                 150                 155                 160

Thr Asp Leu Trp Ala Asp Glu Gln Ile His Tyr Thr Gly Asp Ser Trp
                165                 170                 175

Asn Asp Thr Gly Asn Asn Leu Tyr Gly Asn Phe Lys Gln Phe Leu Leu
            180                 185                 190

Leu Lys Lys Lys Asn Arg Ala Leu Lys Leu Met Leu Ser Val Gly Gly
        195                 200                 205

Trp Thr Phe Gly Pro His Phe Ala Pro Met Ala Ala Asp Ala Lys Lys
    210                 215                 220

Arg Ala Lys Phe Val Ser Thr Ala Ile Thr Ile Leu Glu Asn Asp Gly
225                 230                 235                 240

Leu Asp Gly Ile Asp Ile Asp Trp Glu Tyr Pro Ser Asp Ser Thr Gln
                245                 250                 255

Ala Ala Asn Phe Val Leu Leu Leu Lys Glu Leu Arg Ala Gly Leu Thr
            260                 265                 270

Ala His Gln Ala Lys Lys Asn Glu Thr Asn Pro Tyr Leu Leu Ser Ile
        275                 280                 285

Ala Ala Pro Cys Gly Pro Asp His Tyr Lys Val Leu Gln Val Ala Lys
    290                 295                 300

Met Asp Gln Tyr Leu Asp Phe Trp Asn Leu Met Ala Tyr Asp Phe Ala
305                 310                 315                 320

Gly Ser Trp Ser Ala Leu Thr Gly His Gln Ala Asn Leu Trp Asn Ile
                325                 330                 335

Lys Gly Ala Pro Pro Ser Ala Asp Asp Ser Ile Asn Tyr Tyr Ile Gly
            340                 345                 350

Gln Gly Val Val Ser His Lys Leu Val Leu Gly Ile Pro Leu Tyr Gly
        355                 360                 365

Arg Gly Phe Glu Asn Thr Asp Gly Pro Gln Gln Pro Tyr Arg Gly Thr

370                 375                 380

Gly Gln Gly Thr Trp Glu Ala Gly Asn Trp Asp Tyr Lys Phe Leu Pro
385                 390                 395                 400

Val Lys Gly Ala Lys Glu Met Ile Asn Thr Lys Ile Ala Ala Ser Trp
                405                 410                 415

Ser Tyr Asp Ser Ala Lys Arg Glu Phe Ile Ser Tyr Asp Thr Pro Gln
            420                 425                 430

Asn Val Leu Leu Lys Cys Gln Tyr Ile Arg Asn Lys Arg Leu Arg Gly
        435                 440                 445

Ala Met Phe Trp Glu Leu Ser Gly Asp Ala Thr Lys Ser Gln Gly Gly
    450                 455                 460

Ala Glu Arg Ser Leu Ile Ala Leu Thr Ala Lys Asn Met Gly Thr Leu
465                 470                 475                 480

Asp Ala Thr Leu Asn His Ile Ser Tyr Pro Phe Ser Lys Trp Asp Asn
                485                 490                 495

Val Lys Asn Gly Leu Lys
            500

<210>  3
<211>  305
<212>  DNA
<213>  Ustilago maydis

<400>  3
caagaagaag ttgaagtaag ctgtttcgct tttgctcgat tgcgattcgg atcttttggc      60

tcttggtttc ttctcaacac acacacacac acacacacac acacacacac acacacacac     120

acacacacac acgcacatct acatatatgc aacacatcgc acaccacaca tggcacagta     180

caagcattgc gcctgcgtgc tggagtgcac tggcctcgcg cctacaccca ctggctctga     240

cagcgctcgt ttgtctttgt cagttgtttc aaaaccacat gttattcttg gttgtgccgt     300

ctaga                                                                 305

<210>  4
<211>  792
<212>  PRT

<213>  Ustilago maydis

<400>  4

Met Ser Asp Ser Ile Tyr Ala Pro His Asn Lys His Lys Leu Glu Ala
1               5                   10                  15

Ala Arg Ala Ala Asp Ala Ala Ala Asp Asp Ala Ala Thr Val Ser Ala
            20                  25                  30

Leu Val Glu Pro Thr Asp Ser Thr Ala Gln Ala Ser His Ala Ala Glu
        35                  40                  45

Gln Thr Ile Asp Ala His Gln Gln Ala Gly Asp Val Glu Pro Glu Arg
    50                  55                  60

Cys His Pro His Leu Thr Arg Pro Leu Leu Tyr Leu Ser Gly Val Asp
65                  70                  75                  80

Ala Thr Met Thr Asp Lys Glu Leu Ala Gly Leu Val Phe Asp Gln Val
                85                  90                  95

Leu Pro Val Arg Leu Lys Ile Asp Arg Thr Val Gly Glu Gly Gln Thr
            100                 105                 110

Ala Ser Gly Thr Val Glu Phe Gln Thr Leu Asp Lys Ala Glu Lys Ala
        115                 120                 125

Tyr Ala Thr Val Arg Pro Pro Ile Gln Leu Arg Ile Asn Gln Asp Ala
    130                 135                 140

Ser Ile Arg Glu Pro His Pro Ser Ala Lys Pro Arg Leu Val Lys Gln
145                 150                 155                 160

Leu Pro Pro Thr Ser Asp Asp Ala Phe Val Tyr Asp Leu Phe Arg Pro
                165                 170                 175

Phe Gly Pro Leu Arg Arg Ala Gln Cys Leu Leu Thr Asn Pro Ala Gly
            180                 185                 190

Ile His Thr Gly Phe Lys Gly Met Ala Val Leu Glu Phe Tyr Ser Glu
        195                 200                 205

Gln Asp Ala Gln Arg Ala Glu Ser Glu Met His Cys Ser Glu Val Gly

210                 215                 220

Gly Lys Ser Ile Ser Val Ala Ile Asp Thr Ala Thr Arg Lys Val Ser
225                 230                 235                 240

Ala Ala Ala Ala Glu Phe Arg Pro Ser Ala Ala Ala Phe Val Pro Ala
                245                 250                 255

Gly Ser Met Ser Pro Ser Ala Pro Ser Phe Asp Pro Tyr Pro Ala Gly
            260                 265                 270

Ser Arg Ser Val Ser Thr Gly Ser Ala Ala Ser Ile Tyr Ala Thr Ser
        275                 280                 285

Gly Ala Ala Pro Thr His Asp Thr Arg Asn Gly Ala Gln Lys Gly Ala
    290                 295                 300

Arg Val Pro Leu Gln Tyr Ser Ser Gln Ala Ser Thr Tyr Val Asp Pro
305                 310                 315                 320

Cys Asn Leu Phe Ile Lys Asn Leu Asp Pro Asn Met Glu Ser Asn Asp
                325                 330                 335

Leu Phe Asp Thr Phe Lys Arg Phe Gly His Ile Val Ser Ala Arg Val
            340                 345                 350

Met Arg Asp Asp Asn Gly Lys Ser Arg Glu Phe Gly Phe Val Ser Phe
        355                 360                 365

Thr Thr Pro Asp Glu Ala Gln Gln Ala Leu Gln Ala Met Asp Asn Ala
    370                 375                 380

Lys Leu Gly Thr Lys Lys Ile Ile Val Arg Leu His Glu Pro Lys Thr
385                 390                 395                 400

Met Arg Gln Glu Lys Leu Ala Ala Arg Tyr Asn Ala Ala Asn Ala Asp
                405                 410                 415

Asn Ser Asp Met Ser Ser Asn Ser Pro Pro Thr Glu Ala Arg Lys Ala
            420                 425                 430

Asp Lys Arg Gln Ser Arg Ser Tyr Phe Lys Ala Gly Val Pro Ser Asp
        435                 440                 445

Ala Ser Gly Leu Val Asp Glu Glu Gln Leu Arg Ser Leu Ser Thr Val
    450                 455                 460

Val Arg Asn Glu Leu Leu Ser Gly Glu Phe Thr Arg Arg Ile Pro Lys
465                 470                 475                 480

Val Ser Ser Val Thr Glu Ala Gln Leu Asp Asp Val Val Gly Glu Leu
                485                 490                 495

Leu Ser Leu Lys Leu Ala Asp Ala Val Glu Ala Leu Asn Asn Pro Ile
            500                 505                 510

Ser Leu Ile Gln Arg Ile Ser Asp Ala Arg Glu Gln Leu Ala Gln Lys
        515                 520                 525

Ser Ala Ser Thr Leu Thr Ala Pro Ser Pro Ala Pro Leu Ser Ala Glu
    530                 535                 540

His Pro Ala Met Leu Gly Ile Gln Ala Gln Arg Ser Val Ser Ser Ala
545                 550                 555                 560

Ser Ser Thr Gly Glu Gly Gly Ala Ser Val Lys Glu Arg Glu Arg Leu
                565                 570                 575

Leu Lys Ala Val Ile Ser Val Thr Glu Ser Gly Ala Pro Val Glu Asp
            580                 585                 590

Ile Thr Asp Met Ile Ala Ser Leu Pro Lys Lys Asp Arg Ala Leu Ala
        595                 600                 605

Leu Phe Asn Pro Glu Phe Leu Lys Gln Lys Val Asp Glu Ala Lys Asp
    610                 615                 620

Ile Leu Asp Ile Thr Asp Glu Ser Gly Glu Asp Leu Ser Pro Pro Arg
625                 630                 635                 640

Ala Ser Ser Gly Ser Ala Pro Val Pro Leu Ser Val Gln Thr Pro Ala
                645                 650                 655

Ser Ala Ile Phe Lys Asp Ala Ser Asn Gly Gln Ser Ser Ile Ser Pro
            660                 665                 670

Gly Ala Ala Glu Ala Tyr Thr Leu Ser Thr Leu Ala Ala Leu Pro Ala
        675                 680                 685

Ala Glu Ile Val Arg Leu Ala Asn Ser Gln Ser Ser Ser Gly Leu Pro
    690                 695                 700

Leu Pro Lys Ala Asp Pro Ala Thr Val Lys Ala Thr Asp Asp Phe Ile
705                 710                 715                 720

Asp Ser Leu Gln Gly Lys Ala Ala His Asp Gln Lys Gln Lys Leu Gly
                725                 730                 735

Asp Gln Leu Phe Lys Lys Ile Arg Thr Phe Gly Val Lys Gly Ala Pro
            740                 745                 750

Lys Leu Thr Ile His Leu Leu Asp Ser Glu Asp Leu Arg Ala Leu Ala
        755                 760                 765

His Leu Met Asn Ser Tyr Glu Asp Val Leu Lys Glu Lys Val Gln His
    770                 775                 780

Lys Val Ala Ala Gly Leu Asn Lys
785                 790

<210>  5
<211>  2379
<212>  DNA
<213>  Ustilago maydis

<400>  5
atgtccgatt cgatttacgc cccacataat aagcacaagc tcgaggcagc gcgagccgct      60

gatgctgccg ccgatgatgc cgccaccgtc agcgccctcg tcgagccgac cgactccact     120

gcgcaggcaa gtcacgctgc ggagcaaacc attgatgcgc atcagcaagc aggtgacgtc     180

gaacctgaac gatgccatcc tcacctcact cgccccttgc tctaccttag cggtgtcgat     240

gccaccatga ctgacaagga gctcgccggc cttgtctttg atcaagtact ccccgtccgg     300

ctcaaaattg accgcaccgt gggcgagggc cagacagcca gcggcaccgt cgagttccag     360

acgctggata aggccgagaa ggcttatgct accgttcgtc ctccgatcca gctgcgcatc     420

aaccaggacg cctccatcag agagccgcac ccctcggcca aaccacgtct tgtcaagcag     480

ctgccaccta catcggacga cgcgttcgta tacgacctct tccgtccttt cggtccgctc     540

cgacgagctc agtgtctgct caccaatcca gccggcattc acaccggctt caagggtatg 600
gccgtgcttg aattctactc tgaacaagac gcccagcgtg ccgaatccga gatgcactgc 660
agcgaagtcg gcggcaagtc gatctctgtt gccattgata ccgctacgcg caaggtcagc 720
gctgctgcag ccgagttccg tccctcggct gctgcattcg ttccagcggg cagcatgagt 780
ccgtcagctc cgtcctttga cccttaccct gctggctctc gttccgtttc gacaggctca 840
gccgcttcca tctacgcaac ttctggcgcc gctccgactc acgatacccg caacggtgct 900
cagaagggag cgcgtgttcc gctccagtat tcgagccaag catctacgta tgtcgaccct 960
tgcaacctct tcatcaagaa cctcgacccc aacatggaat ccaatgacct cttcgacacc 1020
ttcaaaaggt ttggccacat cgtctctgca cgcgtcatgc gcgacgataa cggcaagagc 1080
agagaatttg ggtttgtcag cttcaccact cctgacgaag cgcaacaagc tctacaagca 1140
atggacaatg ccaaacttgg caccaaaaag atcattgtcc gtctgcacga gcccaagacg 1200
atgcgtcagg agaagctcgc agcgcgttac aacgctgcca atgccgataa cagtgacatg 1260
agcagcaaca gtccgcccac agaggctcgc aaagccgaca agcgtcaaag cagaagctac 1320
ttcaaagcag gtgtgccttc ggatgccagc ggtcttgtag acgaagagca attgcgctcg 1380
ctcagcactg tcgtgagaaa cgagctcttg tctggcgaat ttacacgaag aatccccaag 1440
gtttcctcgg tcaccgaggc gcagttggac gacgttgtag gcgagctgct cagtctcaag 1500
cttgctgatg ccgtcgaggc gctcaataac ccaatttcgc ttattcaaag gatcagcgat 1560
gctcgcgagc agcttgcaca gaagtctgcc tccactctga cggctccgag tccggcgcca 1620
ctgtctgctg aacacccagc tatgctcggt attcaggctc agcgtagcgt atcaagcgca 1680
tcttcgaccg gcgaaggagg tgcgtcggtc aaggagaggg agaggctact caaggctgtc 1740
atctccgtca ccgaaagtgg tgcccccgtt gaggacatca ccgacatgat cgcaagcctg 1800
ccaaagaagg atcgtgccct tgcgctcttc aatccagagt ttctcaagca aaaagtggac 1860
gaagccaagg acattctcga catcaccgac gaaagtggcg aagatctttc gcctcctcgt 1920
gcttccagtg ggagcgctcc ggtgccattg tcagtgcaga cgcctgcttc tgctattttc 1980
aaagatgcca gcaacggcca gagctccatc tcgcctggcg ctgctgaggc ttacacatta 2040
tccacgcttg ctgcgcttcc tgcagccgag attgtgcgct tggccaactc ccaatccagc 2100
tccggtctgc ctctccctaa agcggatcca gcgacggtca aggcaaccga cgatttcatc 2160
gactcgcttc agggcaaagc agcgcacgat cagaagcaga agctgggtga tcagctcttc 2220
aaaaagatcc gtacgttcgg cgtcaaaggc gcacccaagc tcaccatcca cttgttggat 2280

tccgaagact tgcgggcgct ggcgcatctc atgaactcgt acgaggacgt gctcaaggaa 2340
aaagttcagc acaaggttgc tgcaggtctg aacaagtga 2379